# EUROPEAN PATENT SPECIFICATION

(11) **EP 1 470 224 B1**
(45) Date of publication and mention of the grant of the patent: **12.01.2011**
(21) Application number: 03702707.5
(22) Date of filing: 23.01.2003
(51) Int. Cl.: C12N 15/10

(54) **MOLECULAR LIBRARIES**
MOLEKULARE BIBLIOTHEKEN
BANQUES DE MOLECULES

(30) Priority: 23.01.2002 GB 0201523; 23.01.2002 GB 0201522
(43) Date of publication of application: 27.10.2004
(73) Proprietor: El-Gewely, Mohammed Raafat, 9024 Tomasjord (NO)
(72) Inventor: El-Gewely, Mohammed Raafat, 9024 Tomasjord (NO)
(74) Representative: Rigby, Barbara Nicole
(86) International application number: PCT/GB2003/000296
(87) International publication number: WO 2003/062420

(56) References cited:
- TENSON T ET AL: "ERYTHROMYCIN RESISTANCE PEPTIDES SELECTED FROM RANDOM PEPTIDE LIBRARIES" JOURNAL OF BIOLOGICAL CHEMISTRY, AMERICAN SOCIETY OF BIOLOGICAL CHEMISTS, BALTIMORE, MD, US, vol. 272, no. 28, 1997, pages 17425-17430, XP000910236 ISSN: 0021-9258
- TENSON TANEL ET AL: "Inhibition of translation and cell growth by minigene expression." JOURNAL OF BACTERIOLOGY, vol. 181, no. 5, March 1999 (1999-03), pages 1617-1622, XP002245451 ISSN: 0021-9193
- YANG M ET AL: "PROTEIN-PEPTIDE INTERACTIONS ANALYZED WITH THE YEAST TWO-HYBRID SYSTEM" NUCLEIC ACIDS RESEARCH, OXFORD UNIVERSITY PRESS, SURREY, GB, vol. 23, no. 7, 1995, pages 1152-1156, XP002038707 ISSN: 0305-1048
- GEYER C RONALD ET AL: "Mutagenesis by peptide aptamers identifies genetic network members and pathway connections" PROCEEDINGS OF THE NATIONAL ACADEMY OF SCIENCES OF USA, NATIONAL ACADEMY OF SCIENCE. WASHINGTON, US, vol. 96, no. 15, 20 July 1999 (1999-07-20), pages 8567-8572, XP002193742 ISSN: 0027-8424
- TRIPATHI SHAILA ET AL: "Ketolide resistance conferred by short peptides." JOURNAL OF BIOLOGICAL CHEMISTRY, vol. 273, no. 32, 7 August 1998 (1998-08-07), pages 20073-20077, XP002245452 ISSN: 0021-9258
- PHIZICKY E M ET AL: "PROTEIN-PROTEIN INTERACTIONS: METHODS FOR DETECTION AND ANALYSIS" MICROBIOLOGICAL REVIEWS, AMERICAN SOCIETY FOR MICROBIOLOGY, WASHINGTON, DC, US, vol. 59, no. 1, 1 March 1995 (1995-03-01), pages 94-123, XP000196498 ISSN: 0146-0749

## Description

The present invention relates to molecular libraries, in particular to molecular libraries which can be screened to identify members of the library with certain characteristics.

The complex interactions and pathways between biomolecules in living organisms are the focus for much research, particularly as scientists strive to develop new candidate drugs for the diagnosis, prevention or treatment of disease. Of particular interest are proteins which have functionally active surfaces and pockets which are characterised by the three dimensional shape of the protein molecule and particular reactive amino acid side groups. These proteins are able to interact, associate or bind with a variety of other biomolecules, including other proteins, nucleic acids, organic molecules, ions etc.

There is a great desire to identify molecules which can undergo specific interactions with target molecules, e.g. proteins, such molecules may be able to act as agonists or antagonists of enzyme or receptor activity for example, and more recently it has been proposed that the three dimensional shape of target proteins may be altered through interactions with small peptides (Nature Biotechnology letter El-Gewely 1999, Vol 17, p210). In this way the wild-type function of a mutant protein can be restored.

A variety of molecular libraries which can be screened to identify molecules with desired properties are known in the art. These may comprise organic chemicals synthesised by traditional methods or chemicals produced by combinational chemistry. It is expensive to generate very large libraries of such organic chemicals even using combinatorial techniques and so interest has turned to biological systems.

The best known of these is phage display where peptides are expressed on the surface of a filamentous phage as a fusion protein with a coat protein. Ideally each phage will carry only one such peptide (although it will generally have several copies of that molecule) on its surface and these are then screened *in vitro* for their ability to bind to a target ligand. The phage provide a useful link between genotype and phenotype, as the phage which has a useful ligand on its surface will also have the nucleic acid inside it which codes for that peptide ligand. The phage can be cultured, the nucleic acid amplified and then sequenced to determine the identity of the ligand exhibiting desirable properties. However, only ligands in the form of fusion proteins with coat proteins can be investigated according to this method and it is only possible to perform a rather limited *in vitro* binding assay. Much more useful information could be generated if it was possible to analyse the performance of the members of the molecular library *in vivo*, in an environment similar to that which would be found inside the cells where the molecule is intended to act.

Another biologically based system which provides a molecular library is known as Aptamers/SELEX (Ellington and Conrad 1995, Gold et al. 1997 etc. SELEX stands for Systematic Evolution of Ligands by Exponential Enrichment. According to this technique, single or double stranded nucleic acid molecules that bind to other molecules are investigated, e.g. in order to find a small molecule - in this case a nucleic acid molecule, that binds to a target protein. The short nucleic acid molecules with strong and specific binding capabilities are the "aptamers". Again, these systems only allow *in vitro* screening of molecules.

Thus, there remains a need for a relatively cheap molecular library whose members can be analysed *in vivo* without the need to generate fusion proteins which as a whole may not reflect the potential binding activity of the individual library molecules themselves. These problems have been addressed by the present invention, which according to one aspect provides a library of nucleic acid constructs which each express free peptides in an intra-cellular environment, each of said peptides consisting of the sequence M-G/M/V-(X)ₙ, wherein n is an integer from 3 to 18, M is methionine, G is glycine, V is valine and each X, which may be the same or different, is any genetically encoded amino acid, said free peptides are optionally initially expressed as a fusion peptide with a cleavable signal sequence responsible for localization or translocation of the peptide of formula M-G/M/V- (X)ₙ, said signal sequence being no more than 30 amino acids in length and wherein said library has at least 2000 different members.

Thus, the above defined free peptides are optionally expressed as a fusion peptide with a cleavable signal sequence responsible for localization or translocation of the peptide. Such signal sequences are well known in the art and will typically be no more than 30, preferably no more than 20 amino acids in length. The term 'free' will be understood with this in mind and therefore constructs whose initial translation product incorporates a peptide of formula M-G/M/V-(X)ₙ together with a cleavable signal sequence are still considered 'constructs which can express free peptides' of sequence M-G/M/V-(X)ₙ. The free peptides encoded by the libraries of the present invention can be contrasted with libraries of proteins which comprise peptides of interest fused to a large carrier protein, e.g. designed for surface display of the peptides of interest.

The integer represented by the letter n in the above formula is from 3 to 18, preferably from 3 to 10 and typically from 3 to 5.

Thus in each of the expressed peptides the first residue is methionine, the second residue is glycine, methionine or valine and the third and subsequent amino acids may be any of the genetically coded amino acids. The universally accepted single letter code system for amino acids is used throughout.

It is well known that there are 20 genetically coded amino acids and any of these may be present in the encoded peptides. There will be between 3 and 18 X residues which may be the same or different and it is envisaged that for a given library, once the desired length of the peptides has been set, representatives of most if not all available combinations of residues will be present. Thus for example, using the single letter code for amino acids, where the desired peptides are pentamers, the library would encode most or all of the following peptides, which are themselves only listed by way of example, MMACD, MMACE, MMACF, MMACG, MMADE, MMADF, MMADG, MGACD, MGACE, MCACG, etc. Preferably the second amino acid is glycine.

A library of constructs according to the invention may encode peptides of different lengths but typically a library will encode only peptides of 1-3 different lengths, often of only one length. Thus a library will typically have constructs which encode just pentamers or just hexamers etc. The nucleic acid encoding these peptides are referred to herein as microgenes or forming a microgenex library.

The present invention provides a library of constructs which in turn is capable of generating a library of peptides which can be screened for their activity in a given intra-cellular environment. Thus, according to the present invention, the library of molecules which is actually investigated/screened is made up of peptides, the library as a whole providing a repertoire of different shapes. The "shapes" will also have regions of positive and negative charge and various reactive groups which will impact on their *in vivo* activity. The peptides are putative ligands for one or more target proteins and are conveniently referred to herein as a 'ligand repertoire' when either peptide or corresponding nucleic acid form.

In a further aspect the present invention provides a (synthetic) library of free peptides, each member of said library having the amino acid sequence M-G/M/V- (X)ₙ, wherein n is an integer from 3 to 18, M is methionine, G is glycine, V is valine and each X, which may be the same or different, is any genetically encoded amino acid, each free peptide optionally forming part of a fusion peptide with a cleavable signal sequence responsible for localization or translocation of the peptide of formula M-G/M/V-(X)ₙ, said signal sequence being no more than 30 amino acids in length and wherein said library has at least 2000 different members. The library is synthetic in that it has been generated through the use of recombinant DNA technology and is not naturally occurring.

The libraries of the present invention have at least 50, typically at least 100, preferably at least 500 members more preferably at least 2000. The members of the library are usually screened at the same or substantially the same time, e.g. the library members are in contact with the host cells at the same time or at least screened as part of a series of coordinated multiplex assays. The library may be screened all together or in batches but in that case at least 8, preferably at least 64 or 96 different library members would still be contacted with the host cell population at the same time. The different nucleic acid constructs are recognisably part of a library as they will generally differ only in the region which encodes the peptides of formula M-G/M/V-(X)ₙ defined above; thus the constructs, which are typically plasmid vectors, will contain the same flanking sequences i.e. promoter etc. on either side of the sequence encoding the peptide of interest.

The term "nucleic acid construct" refers to a nucleic acid molecule which contains a coding region for the peptides as defined herein operably linked to a promoter region. The constructs are typically in the form of expression vectors, e.g. plasmid vectors and other expression vectors such as phagemid or lambda expression vectors can also be used. The constructs are designed to allow expression in prokaryotic or eukaryotic host cells and thus a mammalian, bacterial or yeast etc. expression vector is selected as appropriate. Suitable expression vectors for use with different cell types are well known in the art. The library of peptide molecules encoded by the library of constructs can be analysed for their ability to interact with a given target system or protein. If the target protein is mammalian it follows that the library will ideally be expressed in mammalian cells and the expression vector will be selected accordingly.

According to a further aspect, the present invention also provides a method of generating a library of nucleic acid constructs which each express free peptides in an intra-cellular environment, said free peptides consisting of the formula M-G/M/V- (X)ₙ wherein n is an integer from 3 to 18, M is methionine, G is glycine, V is valine and each X, which may be the same or different, is any genetically encoded amino acid, said free peptides optionally being initially expressed as a fusion peptide with a cleavable signal sequence responsible for localization or translocation of the peptide of formula M-G/M/V- (X)ₙ, said signal sequence being no more than 30 amino acids in length, which method comprises synthesising a library of DNA molecules which each include the nucleotide sequence ATGGGA(NNK)ₙ, wherein n is an integer from 3 to 18, N is A, C, T or G and K is G or T and wherein each NNK triplet may be the same or different, and inserting a library of synthesised DNA fragments which each includes a nucleotide sequence of formula ATGGGA(NNK)ₙ into expression vectors, wherein said library has at least 2000 different members.

Thus the invention provides libraries for use in any host cell for which a suitable promoter to drive expression is known. Modifications to nucleic acid libraries disclosed herein which would be necessary for expression in a different host cell system are within the competence of the skilled man, so a range of bacterial, eukaryotic or yeast compatible libraries may readily be developed. The Shine-Dalgarno sequence which is required for expression in bacteria does not hinder expression in eukaryotic cells.

It should be understood that the construct library is capable of generating a repertoire of different molecules with different three dimensional configurations and different functional groups. As with a chemical library, this same library of peptides can be used in many different screening methods; whether it be for allosteric inhibition of an enzyme, the ability to competitively inhibit the activity of an intra-cellular messenger at a receptor binding site, to control nucleic acid transcription or translation, or to restore the function of a mutant protein through interaction therewith which alters the mutant protein's three dimensional shape.

Whereas a phage display library is used in an *in vitro* binding assay, the libraries of the present invention, because the peptides are expressed as free peptides inside the cell, may be used in an *in vivo* screening method to investigate the activities of the peptide ligands in normal cell conditions. Thus toxic molecules may be excluded at an early stage in testing.

The cells in which the library of peptide molecules are to be expressed can internalise the expression vectors into which the ligand repertoire has been constructed and there is thus no need either to synthesize peptides or purify peptide libraries. Preferably, the expression vectors are capable of autonomous replication to prevent the dilution of transfected ligand-encoding plasmids during cell division or the integration of the (e.g. plasmid) vector into the host cell chromosome, which causes difficulties in sequence analysis of the region of the nucleic acid within a cell which encodes the peptide ligand of interest.

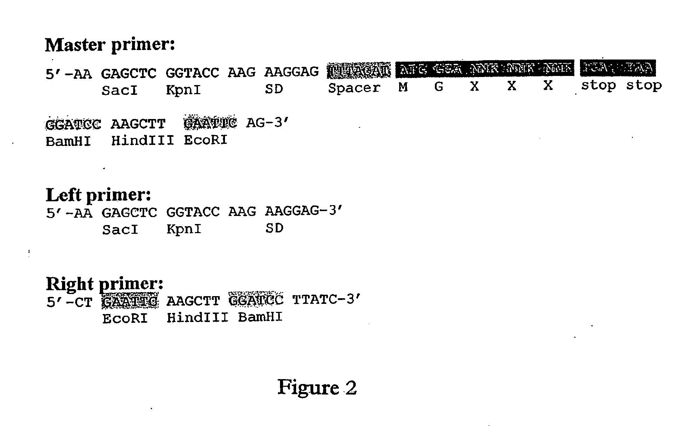

Recombinant technology to generate expression vectors is well known and a suitable method for generating the library of constructs incorporating the desired sequence variation is described in the Examples. The variation in the nucleic acid sequence which translates into the different peptide ligands is typically generated through the synthesis of a master primer which has regions of known sequence flanking the region which encodes the peptide of formula M-G/M/V-(X)ₙ defined above (see Figure 2). As the first amino acid is always methionine, the first three nucleotides will be ATG, in the preferred case where the second residue is glycine, the next three nucleotides will be GGA and the remaining triplets follow the formula NNK, wherein N is A, C, T or G and K is G or T. This can allow for any nucleotide in any of the Y positions.

The primer is generated adding particular nucleotides in turn until the NNK regions are reached when all 4 nucleotides are added in equal molar amounts for the addition of 2 nucleotides to the growing strand and then G and T are added in equal molar amounts for the third position in the triplet. This cycle of additions is repeated until nucleic acid encoding for the peptides of the required length is generated. There are many commercially available machines or services for synthesis of oligonucleotides, e.g. as provided by Sigma and Life Technologies.

This technique provides a simple method for generating a vast range of potentially active biomolecules. The NNK motif of nucleotide addition means that all of the standard amino acids can be incorporated into the peptides in any combination, so the peptides can include all the range of reactive side groups and overall variation in size, shape, polarity, hydrophobicity etc. which are provided by the amino acids individually and by combinations of amino acids in juxtaposition.

In a further aspect, the present invention provides a method of identifying an active peptide ligand of formula M-G/M/V-(X)ₙ as defined above having the ability to restore the function of a target protein, which method comprises:
a) transforming a host cell population with a library of nucleic acid constructs as defined herein;
b) culturing the transformed host cells under conditions suitable for intra-cellular expression of the free peptides of formula M-G/M/V-(X)ₙ;
c) analysing the host cell population to determine the effect of the expressed free peptides on said target protein, which target protein form part of a reporter system; and optionally
d) identifying the peptide of formula M-G/M/V-(X)ₙ in those cells in which the reporter system indicates a positive response.

The restoration of function will typically be the restoration of wild-type activity in the target protein, the host cells having a non-wild-type mutant form of the target protein.

Alternatively viewed, the present invention provides a method of screening a library of peptides of formula M-G/M/V-(X)ₙ wherein n is an integer from 3 to 18, M is methionine, G is glycine, V is valine and each X, which may be the same or different, is any genetically encoded amino acid, which method comprises:
a) transforming a host cell population with a library of nucleic acid constructs which can express free peptides of formula M-G/M/V- (X)ₙ, said free peptides are optionally initially expressed as a fusion peptide with a cleavable signal sequence responsible for localization or translocation of the peptide of formula M-G/M/V- (X)ₙ, said signal sequence being no more than 30 amino acids in length;
b) culturing the transformed host cells under conditions suitable for intra-cellular expression of the peptides of formula M-G/M/V-(X)ₙ; and
c) analysing the host cell population to determine the effect of the peptides of formula M-G/M/V- (X)ₙ on a reporter system, wherein the reporter system includes a target protein and the ability of said peptides to restore the function of said target protein is analysed.

These same method steps could also be considered to define a method of screening a library of nucleic acid constructs. The 'microgenes' encoding the library of peptides may be expressed constitutively or expression can be induced from a promoter which is operably linked to said microgene. The constructs may be autonomously replicated or integrated in the host chromosome.

We describe methods of "screening a library". While the library may have been selected or designed to have certain structural motifs and the nature of individual members of that library can be assumed, this phrase implies that it is not known which member is introduced into which cell (or cell sample) and the molecules are not introduced in a controlled series.

Suitable transformation and culturing techniques are well known in the art and some are discussed in detail in the Examples hereto. Suitable reporter systems are discussed in detail below and will depend on the desired activity of the ligands. The reporter system will conveniently involve up or down regulation of gene expression, either at the level of transcription or translation. The gene whose level of expression is monitored may be native to the host cell but is typically an exogenous reporter gene. The reporter system will incorporate a target protein whose activity can be altered through interaction with one or more members of the library of peptides defined and described above. Binding or association of the peptide ligand to or with the target protein will, in some cases, result in a conformational charge in the target protein and where this results in a change in activity of the target protein this may be observed through the reporter system. Suitable target proteins are discussed in more detail below and include enzymes and transcription factors. The reporter system may be directly or indirectly related to the desired ligand function. For example, if the ability of a ligand to activate transcription or translation of a gene conferring antibiotic resistance is being investigated, culturing the host cells in the presence of that antibiotic would readily identify successful ligands as cells which expressed non-successful ligands would not grow. Given the size of the peptides, activation is unlikely to be through direct nucleic acid binding but through interaction with the protein machinery which performs and/or controls transcription/translation.

Thus analysis of the host cell population may simply be by observation, e.g. of growing plaques or through detecting a particular colour, for example if the β-gal reporter system is used.

In step d) the active peptide is typically identified by analysis of the nucleic acid encoding it. The Examples provide a discussion of how the nucleic acid of the constructs from selected host cells may be sequenced. For a given host cell, the link between phenotype of the peptide ligand in terms of its impact on the reporter system and genotype of the microgene encoding said ligand allows convenient identification of ligands exhibiting the desired activity.

A library of molecular shapes, i.e. ligands, for *in vivo* screening has a wide variety of uses. Of particular interest is the potential for members of a library to encode peptides which can take part in protein therapy through protein function restoration. It has recently been shown that while a mutant protein may have an altered three-dimensional structure due to a non-wild type primary structure, the three-dimensional structure can be corrected without changing the primary structure. Small molecules, e.g. peptides, may be used which interact with the mutant protein to modify its three dimensional structure and thus its activity. Proteins are known to change their conformation on binding or associating with a range of different classes of molecules, e.g. other proteins, nucleic acids, prosthetic groups such as heme, ions such as Ca²⁺ etc.

Peptide ligands identified according to the above described method can be used therapeutically to alter the three-dimensional structure of a mutant protein and thus restore or alter the function or activity of that mutant protein. Typically the ligand can restore, in whole or in part, wild type protein function. Peptide ligands identified according to the above screening method may be chemically modified before being used in therapy, in particular to enhance *in vivo* stability or by introduction of targetting or signal moieties such as the HIV Tat tag or folate, the receptor for which is over-expressed in many cancer cells. There are standard techniques which can be used to achieve this e.g. C terminal modifications such as amidation or N terminal modifications or the use of D- instead of L-amino acids.

It has been found that very small peptides can restore or modify the function of target proteins through interacting with them *in vivo* and causing changes in their conformation. The free peptides expressed by the library of nucleic acid constructs will preferably have only 3-8 amino acids, particularly preferably 3-6 amino acids. If a signal sequence is used, these preferred lengths refer to the peptides generated after cleavage of the signal sequence.

In a further aspect, the present invention therefore provides for the use of a library of nucleic acid constructs as defined herein in a method of screening a library of molecules for the ability of members of that library to restore or modify the function of a target protein in an intra-cellular environment, which method comprises introducing the library into cells which have a reporter system which allows the identification of those cells in which the function of the target protein has been restored or modified.

Thus the reporter system is compatible with, or more particularly includes, the target protein. The reporter system preferably comprises a reporter gene which is operably linked to a sequence of nucleotides which provides a binding site for the target protein or for a protein which associates with or is a substrate for said target protein.

The screening methods of the invention give information about individual members of the library of molecules. The method is performed in an intra-cellular environment (i.e. *in vivo*), and therefore all toxic compounds will be weeded out very early on. This reduces the cost of screening and of pre-clinical and even phase I clinical trials. The method is able to indicate in a qualitative possibly also quantitative manner the performance of individual molecules in the test system. A target protein and suitable host cells are selected and the method designed so that the reporter system is able to give information about the ability of each molecule to restore or modify the function of the target protein.

The reporter system is typically designed to measure restoration of a particular function of the target protein, for example the ability to act as a DNA binding transcription factor. Thus restoration of function refers to a return to wild-type function of the protein in at least one physiologically relevant and measurable respect.

In many cases, the wild-type functions of the target protein in all respects may be restored but this is not essential for the successful working of the invention. It will be appreciated that partial restoration of the function of a protein may still be useful and it is not a requirement for a positive identification according to the claimed screening method to provide 100% of wild-type activity.

The sensitivity of the reporter system may conveniently be modified to provide a more or less stringent assay and thus to identify as giving "positive" results only those molecules which have achieved a significant increase in protein function. Preferably, molecules giving positive results according to the claimed screening methods will have brought about at least a 30%, more preferably at least a 50%, particularly preferably at least a 70% restoration in wild-type protein function, with respect to the particular function monitored by the intra-cellular reporter system. In some instances, a ligand may correct the mutant to a level of measured activity which is actually greater than wild type. It will be clear to the skilled reader that the art provides a number of ways of comparing the relative activities of different molecules. Some reporter systems e.g. those based on fluorescence may be quantitative and thus facilitate such a comparison between wild-type and corrected mutant. However an assessment relative to the wild-type protein may more conveniently be performed as part of a separate test utilising serial dilutions, measurement of plaque size etc.

Suitable target proteins for restoration of function will include those whose presence in mutated form is associated with a disease state. The mutated version of the protein will typically have an altered 3-dimensional structure which affects its ability to interact with other molecules (ions, intra-cellular organelles and other cell components etc.). The members of the molecular library will be screened for their ability to interact with the mutant target and thus alter 3-dimensional structure. The 3-dimensional structure *in vivo* may be closer in one or more respects to the wild-type 3-dimensional structure as a result of successful interaction with a member of the library but protein function may be restored through a further compensating change in 3-dimensional structure which has a return to wild-type function as a 'net' result. Preferred target proteins will be those wherein point mutation(s) are the cause of disease. Particularly preferred are transcription factors (DNA binding proteins) such as p53, but others would be enzymes, peptide hormones or receptor molecules.

All diseases caused by monogenic Mendelian mutations could be targets for treatment with molecules identified according to the screening methods of the present invention. These include genetic (i.e. hereditary) diseases, cancer and symptoms of aging caused by mutations. The host cells should provide a suitable model for determining whether the members of the library are capable of causing the desired change in target protein function with a view, typically, to therapeutic administration of successful members of the library or a derivative thereof.

Thus, where protein function restoration is required, the host cells will typically be derived from cancerous or other diseased cells and naturally produce the dysfunctional target protein. Suitable cell lines are available, for example in cell culture collections such as the ATCC and may be derived from osteosarcoma, adenocarcinoma etc., or indeed any established cell line that expresses the mutant protein. Cell lines that lack any expression of the protein of interest can also be used after introducing and expressing the gene encoding the mutated protein.

Standard transfection techniques may be used to introduce the nucleic acid constructs into the host cells in which expression of the free peptide ligands is to take place. The terms 'transformation' and 'transfection' are used interchangeably herein.

The appropriate reporter system for a given screening method will depend upon the nature of the target protein under investigation. The reporter system is chosen to be responsive to the target protein and thus be capable of indicating the functional status of the target protein. The mammalian p53 protein illustrates the principle behind a suitable reporter system and is a particularly preferred target protein. P53 functions in mammalian cells mainly through the ability to act as a transcription factor and is a transcriptional activator of several genes associated with cell cycle regulation such as p21, Bax, CD95(Fas/Apo-1) and 4-3-3σ (HME1). It can also down regulate the transcription of cell-cycle-regulating genes such as Bcl2, Cdc2 and cyclin. P53 acts as a checkpoint, monitoring DNA damage and regulating cell cycle progression. Loss of p53 activity predisposes cells to the acquisition of oncogenic mutations and may favour genetic instability - 90% of mutations reported in the p53 mutation database are found in the DNA binding domain. Mutations in p53 can lead to cancer formation where p53-mediated apoptosis is deficient due to the mutation.

P21 is one of the p53-transactivated genes that are critical in cell cycle control and many p53 mutants found in cancer cells lose the ability to transactivate p21 transcription. Thus the transactivation level of p21 promoter in a cell reflects whether the p53 protein expressed in that cell has wild type function. As discussed in more detail in the Examples, a p53 reporter system was constructed by cloning human p21 promoter upstream of the puromycin resistance gene. The reporter construct can provide human cells with puromycin resistance only in the presence of wild type or wild-type functioning p53 that transactivates the p21 promoter. Such a reporter system offers significant practical benefits as a life-death selection system is possible where only cells with wild-type-functioning p53 can survive when grown in the presence of puromycin.

Thus, while the host cells may have a reporter system as part of their normal genome which can be utilised, typically the host cells will have been modified to include a suitable gene based reporter system. The genetic constructs which comprise all or part of the reporter system will thus have been introduced into the host cells, e.g. by standard transformation/transfection techniques, before the screening method is performed. In certain circumstances, the reporter constructs could be introduced at the same time as the molecular library or even, but not preferably, after introduction of the library. Thus for performance of the screening methods of the invention, host cells will preferably have been co-transfected (although not necessarily simlutaneously) with a reporter construct and a construct which encodes a peptide whose ability to restore or modify the function of a target protein is to be investigated. Alternatively, members of a chemical compound library may be contacted with the host cells which have been transfected with reporter constructs.

Target proteins are preferably DNA binding proteins which up or down regulate the expression of other genes through binding to promoter or enhancer regions. Their native gene targets may be utilised to report on whether a target protein function has been restored or modified, but preferably their target DNA binding regions will be operably linked to reporter genes such as genes conferring antibiotic resistance, or which encode fluorescent proteins such as GFP (green fluorescent protein), or the much used bacterial reporter enzymes β-galactosidase (β-Gal) or β-glucuronidase (β-Gus) etc. According to a particularly convenient and preferred method the protein products of these reporter genes (e.g. β-Gal or β-Gus) could be fused to a signal peptide in order to 'display' the proteins on the cell surface. In this way, the cells expressing the reporter protein can readily be separated physically from other cells that do not express the reporter protein, e.g. by the use of antibodies. Suitable signal peptides are described in the literature and include the signals from a protein such as PDGFR (platelet-derived growth factor receptor). A typical construct would thus be as follows:
Secretion signal peptide - reporter protein (eg. *β-*Gal/β-Gus) - transmembrane domain; e.g.
METDTLLLWVLLLWVPGSTGD - β-Gal/β-Gus - AVGQDTQEVIWPHSL PFKVWISAILALVVLTIISLIILIMLWQKKPR - stop.

Non-DNA binding proteins can also be used as target proteins provided their wild type activities can be monitored and assayed for or the reporter system engineered such that cell survival depends on restoration of the protein function.

Identification of cells in which the function of the target protein has been restored or modified(e.g. wild-type function has been restored) will depend on the reporter system used. As described above, this is particularly conveniently performed when cells in which wild-type function has not been restored do not survive in the selected culturing environment. Fluorescence detection can also be used to identify cells in which the function of the target protein has been restored or modified. The target protein may, for example, be an enzyme rather than a nucleic acid binding protein and the ability of the target enzyme to act on a substrate may be used as the intra-cellular reporter system allowing identification of cells in which the function of the target protein has been restored or modified. Other suitable reporter system such as the β-gal. blue/white system are well known in the art and can be used or adapted by the skilled man in his chosen screening method.

A library of nucleic acid constructs according to the present invention is conveniently referred to herein as a "Microgenex" library and the stretches of nucleic acid which encode the peptide ligands of interest as "Microgenes".

A further discussion relevant to the context and appreciation of the present invention is found in our co-pending International application filed 23 January 2003 and claiming first priority from GB 0201522.0.

The invention will now be described in more detail in the following non-limiting Examples and with reference to the figures in which:

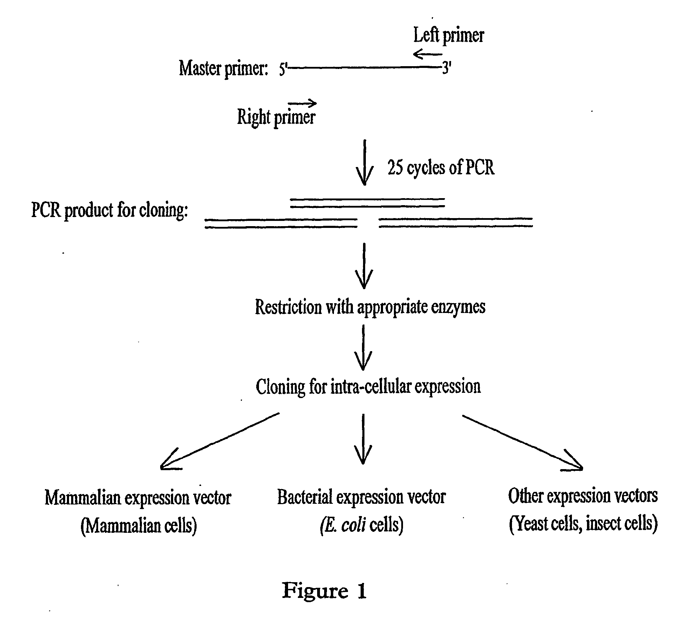

- Figure 1: shows in schematic form the general strategy for construction of a library of nucleic acid constructs according to the present invention.
- Figure 2: illustrates the strategy of primer design for construction of a Microgenex library. Restriction enzyme sites within primers as well as the encoded amino acids are shown below the primer sequences. M = methionine, G = glycine and X = any of the 20 genetically coded amino acids.

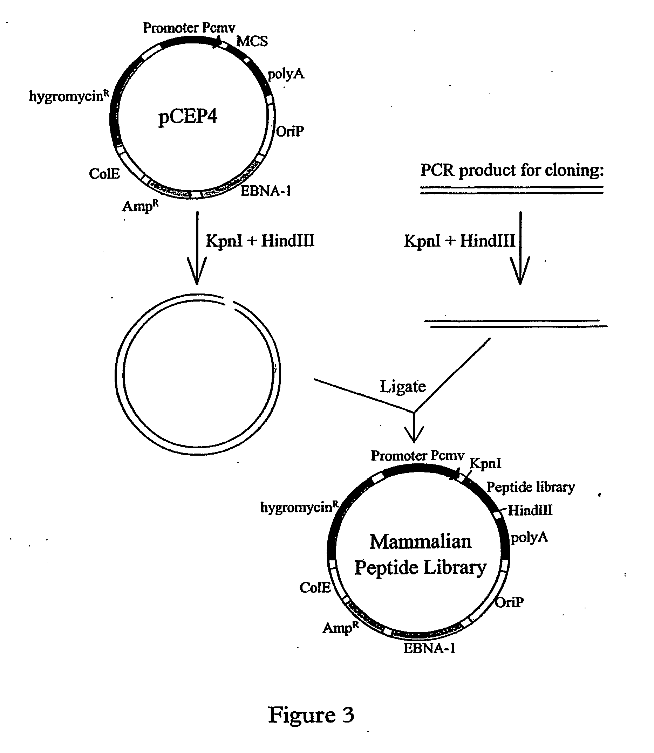

- Figure 3: illustrates the construction of a peptide library for expression in mammalian cells.

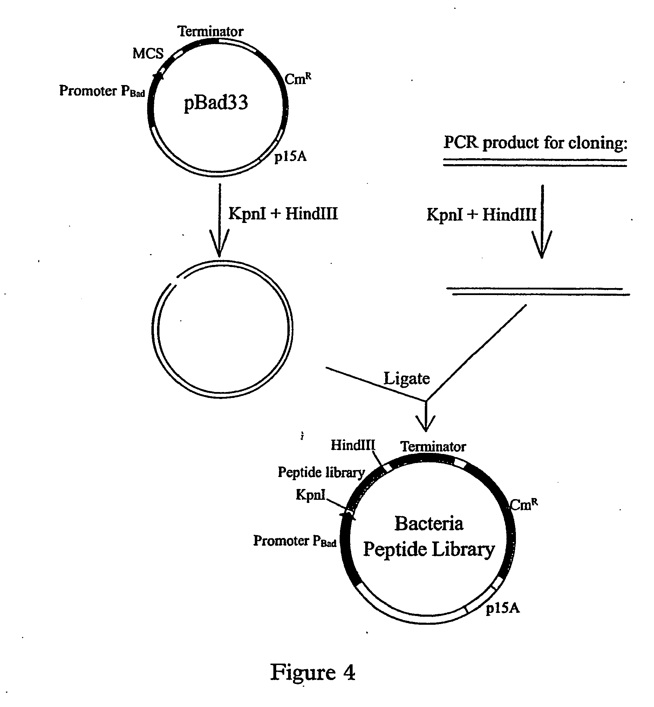

- Figure 4: illustrates the construction of a peptide library for expression in bacteria *(E. coli).*

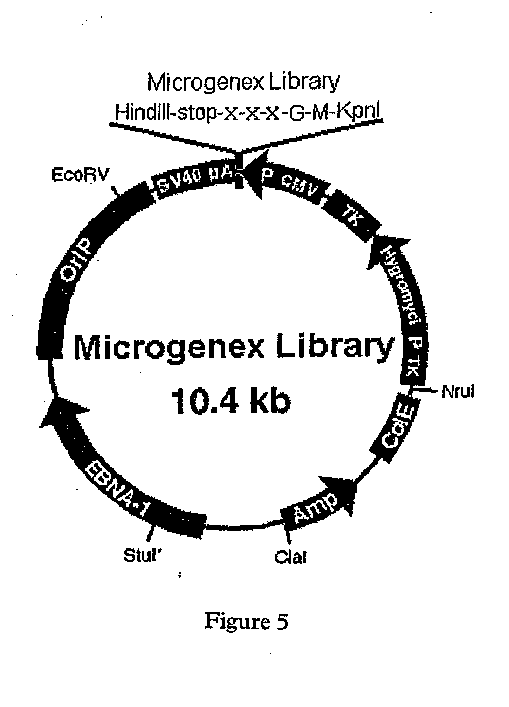

- Figure 5: gives a map of the constructed Microgenex library of the Examples that can be used for expression in mammalian cells.

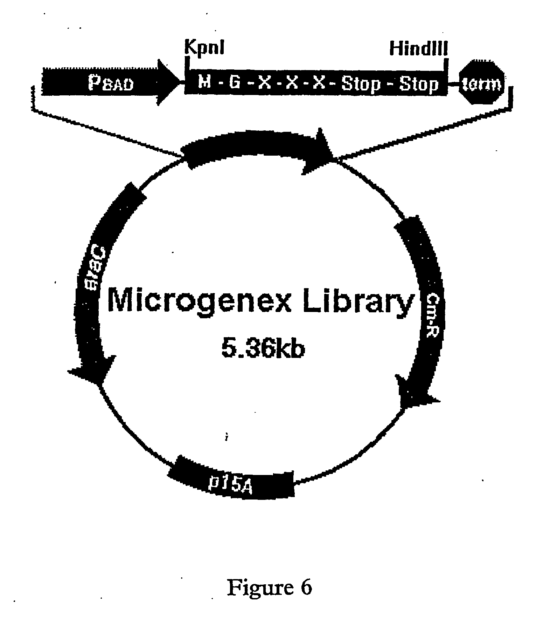

- Figure 6: gives a map of the constructed Microgenex library of the Examples that can be used for expression in *E. coli*. "Cm-R" refers to chloramphenicol resistance gene. "p15A" is replication origin compatible with several other replication origins used in molecular biology and biotechnology. P_{BAD} promoter is suppressed by araC but can be induced by L- arabinose. Peptide library coding sequence was inserted under the P_{BAD} promoter between KpnI and HindIII sites. SD sequence is a ribosome- binding site to help in the translation of the peptides in *E. coli*. The first two amino acids at the N-terminal ("M" for Met, and "G" for Gly) can stabilize the peptides inside cells. The two stop codons assure the termination of translation while the terminator ("term") sequence will terminate the transcription driven by the P_{BAD} promoter. The DNA fragment of the peptide library was generated by PCR, using Master, Right and Left primers as described in the Examples.

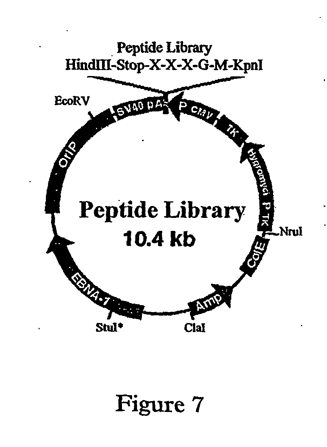

- Figure 7: provides a schematic representation of the generated peptide library plasmid. Each peptide in the library is composed of three random amino acids after two N-terminal amino acids Methioline (Met, or M) and Glycin (Gly, or G). The next three X stands for any amino acid that forms a library consisting of randomly distributed three-amino-acid peptides. The peptide-coding DNA-fragment was inserted into the mammalian expression vector pCEP4 between its KpnI and HindIII site under the CMV promoter. The plasmid has OriP that enables the replication of the plasmid inside mammalian cells. Selection marker expression cassette in the plasmid provides the plasmid- transfected cells with hygromycin resistance.

### Examples

### Example 1: Strategy for Microgenex (nucleic acid library) construction:

### Materials and Methods:

### 1- Primers.

All primers used to construct Microgenex libraries are listed in Table 1.

**Table 1. Primers: Primers used to construct Microgenex libraries.**

| **No** | **Name** | **Sequence** | **bases** |
|---|---|---|---|
| 1 | Master | AAGAGCTCGGTACCAAGAAGGAGTTTACATATGGGANNKN NKNNKTGATAAGGATCCAAGCTTGAATTCAG | 71 |
| 2 | Left | AAGAGCTCGGTACCAAGAAGGAG | 23 |
| 3 | Right | CTGAATTCAAGCTTGGATCCTTATC | 25 |

Primers used for DNA sequence are listed in Table 2.

**Table 2. The Sequencing primers used for the Microgenex verifications.**

| **No** | **Name** | **Sequence** | **bases** |
|---|---|---|---|
| 1 | pCEP-f | AGAGCTCGTTTAGTGAACCG | 20 |
| 2 | pCEP-r | GTGGTTTGTCCAAACTCATC | 20 |
| 3 | Left* | AAGAGCTCGGTACCAAGAAGGAG | 23 |
| 4 | Right* | CTGAATTCAAGCTTGGATCCTTATC | 25 |

| | | | |
|---|---|---|---|
| *These primers can also be used to verify sequence of any Microgenex library. Other primers within the used cloning vector can also be used. | | | |

### 2- Cloning Vectors/ plasmids

pCEP4 (Invitrogen): for mammalian expression.
pBAD33 (Dr. Beckwith, Harvard; Guzman et al., 1995 J. Bacteriol. 177(14) 4121-4130) for *Escherichia coli* expression.

### 3- Escherichia coli strain used for Transforming Constructed Microgenex libraries:

The Electrocompetent DH10B^{™} (Life Technologies, GibcoBRL) was used to transform the ligated Microgenex expression libraries.

### 4- PCR conditions for Microgenex amplifications

### 4.1. Concentrations of primers:

| | |
|---|---|
| Master primer | 25 PM |
| Right primer | 100 PM |
| Left primer | 100 PM |

### 4.2. PCR reactions:

At least two different Polymerases gave good results: Taq polymerase (Life Technologies, GibcoBRL) and rTth DNA polymerase (Perkin Elmer).

### 4.2.1. Standard Taq polymerase as for example in SuperMix (Life Technologies, GibcoBRL).

Prepare 1 to 10 PCR tubes with the following:
90 µl SuperMix
2 µl Master Primer (25 PM)
4 µl Right Primer (100 PM)
1 Left Primer (100 PM)

### 4.2.2 The XL PCR, extra long (rTth DNA polymerase) (Perkin Elmer)

Also this PCR polymerase worked very well after adding buffers according to the manufacturer, but using primers as above and also the PCR cycles as indicated below.

### 4.2.3. PCR Cycles

Best Conditions were found by making a hot start at 95 for 3-5 minutes. PCR cycles were preformed only using 25 cycles. Example for PCR Cycle:

| | | |
|---|---|---|
| Hot start | 95 °C | 3 min. |
| | | |
| 25 cycles | | |
| Denaturation | 94 °C | 1 min. |
| Aneaing | 38 °C | 1 min. |
| Elongation | 60 °C | 1 min. |
| | | |
| Hold at | 4 °C | |

### 5. Ligation of PCR products to the corresponding expression vector:

PCR product was first treated with phenol-chloroform, chloroform and then precipitated with 3 volumes of cold ethanol. The DNA pellet was washed with cold 70% ethanol, dried and dissolved in appropriate volume of H₂O. DNA was then restricted by KpnI and HindIII. Restricted DNA was gel-purified and subsequently ligated to the corresponding expression vector that has been also restricted with by KpnI and HindIII. Ligation mix was precipitated and resuspended in H₂O and subsequently used to transform, by electroporation, of the *Escherichia coli* strain DH10B^{™} (Life Technologies, GibcoBRL). Plating was done on LB ampicilin plates (200 µg/ml) for pCEP4, or chloramphenicol (40 µg/ml) for pBAD33.

### 6. Pooling of colonies:

Colonies from all the plates were pooled together, washed with LB with ampicilin (200µg/ml) if the cloning vector contains beta-lactamase gene, such in the case of pCEP4. This step is important to get rid of cells that lack the plasmid. Pooled cell mixture was divided into small aliquots and stored at -70°C. Each aliquot contained millions of cells to ensure complexity of the Microgenex library.

### 7. DNA Isolation of Microgenex library:

One frozen portion can be used to inoculate a one-liter culture of LB containing the appropriate antibiotic. Standard plasmid DNA isolation protocols/kits are used to isolate the DNA suitable to transform, for example mammalian cells for ligand screening.

Master primer was used as a template for all constructed peptide expression libraries (Microgenex) whether the expression in mammalian cells or *Escherichia coli.* It could also be used for yeast or insect cell expression. Left and Right oligoes were used as forward and reverse primers, in order to generate double-stranded DNA fragments encoding repertoire of peptide library by PCR. The first ATG in this fragment encodes Met as the translation initiation site of the peptides. Gly, the second amino acid encoding by the DNA fragment, assures the peptides expressed to be stable inside cells according to the N-end rule. The following three repeats of NNK code (N for A, C, G, and T in equal molar ratio, K for G and T in equal molar ratio) for all possible amino acids thus form the peptide library. Two stop codons, TGA and TAA, are added right after the last NNK to stop peptide translation. The 5'-end KpnI site and 3'-lend HindIII allows the fragments to be cloned into pCEP4 vector in the correct direction under CMV promoter.

The peptide-coding DNA-fragment was inserted into the mammalian expression vector pCEP4 between its KpnI and HindIII site under the CMV promoter. The plasmid has the replication origin, OriP, which enables the replication of the plasmid inside mammalian cells. Selection marker expression cassette in the plasmid provides the plasmid-transfected cells with hygromycin resistance.

After ligation of the KpnI/HindIII restricted PCR product, and the KpnI/HindIII restricted pCEP4 or pBAD 33, the DNA were electroporated into DH10B^{™} (Life Technologies, GibcoBRL) cells. Transformants were plated on LB plates with ampicilin (200µg/ml) or Chloramphenicol (40 ug/ml) depending on the coloning vector used in the construction of the Microgenex library. All colonies from all the plates were pooled together, washed with LB with ampicilin (200µg/ml), only if the cloning vector contains beta-lactamase gene, such in the case of pCEP4. Pooled cell mixture was divided into small aliquots and stored at -70°C. Each frozen portion of the Microgenex library can be used to inoculate and start a 1-liter-culture (with the appropriate antibiotic, in order to prepare the plasmid DNA. Insertions were verified by PCR of the generated Microgenex with Right and Left oligoes. Also, the forward and reverse primers of pCEP4were used for verifications for the constructed mammalian Microgenex. Similar PCR reactions were preformed using plasmid DNA prepared from a small number of randomly-picked colonies in order to estimate the cloning efficiency and the ratio of peptide-encoding plasmids to empty vectors in any given Microgenex. Note that all generated peptides will start with M and G amino acids followed by three random amino acids (X, X, X) It should be noted that the number of the random amino acids (X) can be changed by changing the number of the codes (NNK).

### Results

Colonies in the range of 25000-75,000 on the plates were commonly obtained ensuring complexity/diversity of the cloned Microgenex repertoire. They were collected into 10 - 12 portions.
Summary of Microgenex libraries are listed in Table 3.

**Table 3. Plasmids used for the construction of microgenex libraries**

| Plamid Name | Promoter | Insertion | Selection marker | Replication origin | Reference. |
|---|---|---|---|---|---|
| pCEP4 | CMV | No | *E. coli*: Amp, Mammalian: library Hygromycin B | *E*.*coli*: ColE, Mammalian: OriP | Vector from Invitrogen |
| Mammalian microgenex library | | Microgenex with amino acid sequence as: M-G-X-X-X. | | | This work, Fig.2.3. |
| pBAD33 | pBAD | No | Chloramphenicol | p15A | Vector from Beckwith; Guzman, et al.,1995 |
| *E. coli* microgenex library | | Microgenex library with amino acid sequence as: M-G-X-X-X. | | | This work. |

Plasmid prepared from one of such frozen aliquots was examined by PCR. When mammalian Microgenex library DNA was used as a template in PCR reactions using pCEP4 forward and reverse primers showed that there were insertions with expected size. PCR results from the 6 randomly picked colonies from the original mammalian Microgenex library plates showed that 4 of the colonies had the right construction (showed correct size of insertion) while two did not.

A test for the presence of Microgene insertions in a constructed mammalian library by PCR from 6 randomly picked colinies indicated that 4 out of the 6 colonies have the right size of insertion. Also, PCR results from 6 randomly picked colonies from the original *E. coli* Microgenex library plates showed that 4 of the colonies had the right construction, in that they showed the correct size of insertion.

### Example 2: Demonstrating the generation and use of a microgene library in identifying a protein binding ligand of interest

### A - Construction of p53 reporter working in mammalian cells

The dysfunction of mutant p53 in cancer cells can result from not only the inability in binding to its specific recognized consensus DNA sequence, but also the mal-localization inside cells or incompetent to interact correctly with other transcription co-factors. The sub-cellular localization mechanism and transcription machinery in mammalian cells is different from that in prokaryotic organisms. The loss of specific DNA binding ability may engage the post-translational modification processes that are totally different from that in prokaryotic organisms. Thus a p53 reporter for mammalian cells has more advantages than that for prokaryotic cells.

P53 protein functions in mammalian cells mainly through its transcriptional factor activity. P21 is one of the p53-transactivated genes that are critical in cell cycle control. P53 can induce cell cycle arrest in G1 phase via transactivation of p21 gene once the cells are under stresses such as DNA damaging. Lots of p53 mutants found in cancer cells lose the ability to transactivate p21 transcription. This leads to not only the vast development of cancer, but also the resistance to cancer chemotherapy and radiotherapy that aim to damage DNA in order to kill cancer cells. Thus the transactivation level of p21 promoter in a cell reflects whether the p53 protein expressed in that cell has wild type function or not.

We constructed our p53 reporter by cloning human p21 promoter upstream of the puromycin resistant gene. It has been reported that basal transcription level from p21 promoter is very low. The reporter can provide human cells puromycin resistance only in the presence of wild type or wild-type-functioning p53 protein that transactivates p21 promoter, while leaving the cells sensitive to puromycin with dysfunctional mutated p53 protein. The advantage to use puromycin resistant gene is to set up a life-death selection: only the cells with wild-type-functioning p53 protein can survive from puromycin selection.

### Materials and Methods

Plasmid and vectors used in this Example are WWP-Luc (gift from Prof. Stanbridge E.J.), pPUR (Clontech), and pUC18 (Pharmacia). Enzymes used for cloning are EcoRI (Promega), PvuII (Promega), SalI (Promega), SphI (Promega), T4 DNA ligase (New England BioLab), Klenow (Promega), and SAP (Shrimp Alkaline Phosphotase). Cell lines for the validation of our reporter are U-2 OS cell (ATCC Number: HTB-96, Homo sapiens (human), osteosarcoma, bone, wild type p53), Saos-2 cell (ATCC Number: HTB-85, *Homo sapiens* (human), osteosarcoma, bone, p53 null), and SW480.7 (ATCC number: CCL 228, *Homo sapiens* (human), colorectal adenocarcinoma, colon, p53 mutant: R273H/ P309S). Medium and other chemicals include McCoy's medium, DME medium, MEM, FCS, calcium phosphate transfection reagents (0.1xTE (pH8.0), 2xHBS (pH7.4), 2M CaCl₂, 15% glycerol in HBS), PBS, and puromycin (Sigma).

WWP-Luc plasmid was restricted by EcoRI and SalI. The 2366bp fragment was gel-purified (1% agarose in 1x TBE) by freeze-thaw method. Vector pUC18 was restricted by EcoRI and SalI, dephosphorylized by SAP, and purified by phenol-chloroform. Then the p21 promoter fragment was subcloned into pUC18 vector by T4 DNA ligase, generating a plasmid named as p21uc. All drug resistant markers in the constructed plasimds, are listed in Table 1. Genes involved in the resistance are also listed.

**Table 1. Drug resistant markers in constructed plasmids and their genes.**

| | |
|---|---|
| Drug | Resistant gene |
| Puromycin | Puromyain-N-acetyl-transferase *(pac)* |
| Neomycin | Neomycin phosphotransferase gene |
| Hygromycin | B Hygromycin B phosphotransferase |
| Histidinol | Histidinol dehydrogenase (hisD) |

P21 promoter was cut out from p21uc first by EcoRI, blunted with Klenow and dNTP, and then by SphI. The 2450bp-p21-promoter fragment was gel-purified using the same method as above. Vector pPUR was restricted fully by PvuII and SphI, dephosphorylized by SAP, and gel-purified by the same method, which removed SV40 early promoter upstream of puromycin resistant gene. Finally the p21 promoter was cloned into pPUR vector upstream of puromycin resistant gene by T4 DNA ligase, generating the p53 reporter named as p21ur.

Verification of the construction was carried out by restriction of the generated plasmid with HindIII. The original vector pPUR has only one HindIII site thus gives one band of 4257bp after gel electrophoresis. This HindIII site was removed while constructing the reporter p21ur. Two new HindIII sites were introduced into p21ur with the p21 promoter thus can give two bands, one at 2.4kb and the other at 3.9kb.

Validation of the responsiveness of p21ur to p53 was carried out by transfection of p21ur to osteosarcoma cell line U-2 OS that expresses wild type p53 protein, Saos-2 that is p53 null, and colon cell line SW480.7 that expresses R273H and P309S double mutated p53 protein. One day before transfection, 3x10⁵ U-2 OS, 1.5 x10⁵ Saos-2, and 3x10⁵ SW480.7 cells were seeded to each well of a 6-well plate. Transfection was carried out by the calcium phosphate method, with 25µg of p21ur for each well. A 2-minute glycerol-shock was applied 3 hours after DNA was added to the cells and the transfected cells were incubated in MEM/10%FCS at 37°C with 5% CO₂ overnight. The transfected cells were transferred to new 6-well plates at the concentration of 5x10⁴ cells per well on the next day, and incubated in growth medium (McCoy's medium/10%FCS for U-2 OS and DME medium/10% FCS for Saos-2 and SW480.7) with puromycin. The same puromycin treatment was also given to the non-transfected cells as a control. Refreshed the medium with puromycin every three-day and observed growth status of the cells.

The cloned vector was restricted by HindIII, which proved that there is the p21 promoter, a ^{~}2.4kb fragment, inserted upstream of the puromycin resistant gene. DNA electrophoresis showed that the restriction gave two bonds with correct theoretical sizes at 2.4 and 3.9 Kb.

Transfection of the reporter p21ur into U-2 OS cell line provided puromycin resistance to the transfected cells (Table 2). Cells transfected with p21ur kept alive after 5 days of puromycin treatment at 0.5µg/ml, while the untransfected cells died out. The transfected cells could not survive from higher concentration of puromycin because of the low expression level of p53 protein. Neither transfected Saos-2 cells (null p53) or SW480.7 cells (mutant p53) survived after 5 days of puromycin treatment at the concentration of 0.5µg/ml or higher, which proved that the resistance of p21ur-transfected U-2 OS cell to puromycin requires the existence of wild type p53 that transactivates p21 promoter. Thus it validated the proper responsiveness of the reporter p21ur to p53 in human cells.

**Table 2. Validation of the reporter p21ur - its response to wild type p53 protein.**

| Puromycin (µg/ml) | Number of cells attached to the bottom of the well after 5 days of puromycin treatment | |
|---|---|---|
| | Untransfected U-2 OS | Transfected U-2 OS |
| 0 | Growing well | 12600 |
| 0.5 | 0 | 11500 |
| 1 | 0 | <5000 |
| 2 | 0 | <5000 |
| 3 | 0 | <5000 |

### B - Construction of peptide library expression in mammalian cells

We have constructed a microgene library to provide a series of molecular shapes for testing. This 'microgenex' approach enabled us to screen among the expressed peptide repretoire and identify some that could correct mutated p53 and could restore all of its downstream activity.

Selection of the functional peptides can be carried out either *in vivo* or *in vitro* according to the peptide library. The advantage of *in vivo* selection over *in vitro* selection is that it allows the peptides to carry out their function in an environment similar or even exactly the same as where they will later be applied in therapy. This makes the peptides more applicable and simplifies the modification step for the selected peptides. Besides, it can often detect weak and transient interactions.

Expression of the peptide library allows *in vivo* screening for possible peptide ligands that can adjust mutant p53 protein back to its wild type function. Once the peptides were constructed into an expression vector, they were easily internalized by cells. With the help of positive-selection reporters, the possible ligands were also readily identified by the recovery of ligand-encoding plasmids from surviving cells. Moreover, according to this technique, there is no need to either synthesize peptides or purify peptides, which can be expensive and labour intensive. An un-integrated, autonomously replicated mammalian expression vector was chosen to prevent the dilution of transfected ligand-encoding plasmids during cell division, or the integration of the plasmids into the host chromosomes, which causes difficulties in rescuing ligand from surviving cells.

### Materials and Methods

pCEP4 (Invitrogen): 
TTGACGCAAA TGGGCGGTAG GCGTGTACGG TGGGAGGTCT ATATAAGCAG AGCTCGTTTA
CAAT TATA pCEP Forward
 
GTGAACCGTC AGATCTCTAG AAGCTGGGTA CCAGCTGCTA GCAAGCTTGC TAGCGGCCGC
primer KpnI HindIII
TCGAGGCCGG CAAGGCCGGA TCCAGACATG ATAAGATACA TTGATGAGTT TGGACAAACC
EBV Reverse primer
ACAACTAGAA
Oligo REGP-10: 
5′-AAGAGCTCGG TACCAAGAAG GAGTTTACAT ATG GGA NNK NNK NNK TGA TAA
KpnI M G X X X stop stop
GGATCCAAG CTTGAATTCA G-3′
HindIII
Oligo REGP-11: 
5′-AAGAGCTCGGTACCAAGAAGGAG-3′
KpnI
Oligo REGP-12: 
5′-CTGAATTCAAGCTTGGATCCTTATC-3′
HindIII
pCEP Forward primer:
   5'-AGAGCTCGTTTAGTGAACCG-3'
EBV Reverse primer:
   5'-GTGGTTTGTCCAAACTCATC-3'

### KpnI, HindIII, T4 DNA ligase, 10xT4 DNA ligase buffer

Oligo REGP-10 was used as a template, while oligos REGP-11 and REGP-12 were used as forward and reverse primers, to generate double-stranded DNA fragments encoding a degenerated peptide library by PCR. The first ATG in this fragment encodes Met as the translation initiation site of the peptides. Gly, the second amino acid encoding by the DNA fragment, assures the peptides expressed are stable inside cells according to the N-end rule. The following three repeats of NNK (N for A, C, G, and T in equal molar ratio, K for G and T in equal molar ratio) code for all possible amino acids and thus form the peptide library. Two stop codens, TGA and TAA, are added right after the last NNK to stop peptide translation. The 5'-end KpnI site and 3'-end HindIII allows the fragments to be cloned into pCEP4 vector in the correct direction under CMV promoter.

After ligation of KpnI/HindIII restricted PCR product and KpnI/HindIII restricted pCEP4, the DNA molecules were electroporated into XL1-blue cells and plated on LB plates with ampicilin. All colonies from the plates were poured together, delivered into small portions and stored at - 70°C. Each portion of the transformed cells can be inoculated and start a 1-liter-culture in order to prepared the plasmids by Qiagen Maxi-Prep kit. Insertions were verified by PCR of the generated plasmids with oligos REGPD-11 and REGPD-12. Same PCR reactions were also applied to plasmids prepared from 6 randomly picked colonies so as to estimate the cloning efficiency and the ratio of peptide-encoding plasmids to empty vectors in the population. Note that all generated peptides will start with M and G amino acids followed by three random amino acids.

### Results

There were ~25000 colonies on the plates. They were collected into 12 portions. Plasmid prepared from one of such portions was examined by PCR.

Gel electrophoresis showed that there were insertions with correct size. PCR results from the 6 randomly picked colonies from the original library plates showed that 4 of the colonies had the right construction (showed correct size of insertion) while two did not. The structure of the generated peptide library plasmid is shown in Figure 7.

### C - Screening for ligands that can correct p53 mutants in SW480.7 cells

SW480.7 is a human colon cancer cell line that carries R273H and P309S double mutations in its p53 gene. R273H is one of the most frequently occurred p53 mutations that have been observed in various cancer tissues. It has been reported that the p53 mutant in SW480.7 loses its function in transactivating its downstream genes such as p21, Bax, 14-3-3-3σ, and CD95. Our purpose of screening the peptide library constructed above is to find out one or more peptide ligands that can restore wild-type p53 function without alteration of the mutant p53 gene in the cell and disturbing cells with wild type p53. The choice of a human cancer cell line provides the real human environment in which the selected peptides can be modified, degraded, and transported to subcellular organelles, and thus assures the reliability of the selected peptides to function in human body.

### Materials and Methods

Human colon cancer cell line SW480.7 with p53 mutations R273H and P309S (ATCC Number: CCL-228, Homo sapiens (human), adenocarcinoma, colorectal; colon), DMEM medium, FCS, PBS, trypsin, puromycin (sigma), hygromycin (sigma), Peptide library (constructed by our laboratory described as above), pCEP4 vector (Invitrogen), calcium phosphate transfection reagents (0.1xTE (pH8.0), 2xHBS (pH7.4), 2M CaCl₂, 15% glycerol in HBS).

DNA extraction reagents (TBS buffer (0.8% NaCl, 0.02%KCl, 0.3%Tris, pH7.4), TE buffer (pH8.0), DNA extraction buffer (10mM TrisCl, 100mM EDTA, 20µg/ml RNase, 0.5% SDS, pH8.0), Proteinase K (18mg/ml), chloroform, phenol (Tris saturated), ethanol, 7.5M ammonium acetate (pH 7.4)), XL1-blue electroporation competent cell, pCEP Forward primer, EBV Reverse primer, dye-terminator thermocyclic DNA sequencing kit, ALF express DNA automatic sequencing system.

The peptide library was transfected into SW480.7 using the calcium phosphate method described in the reporter constructing part. Meanwhile pCEP4 was also transfected into the SW480.7 cells as a negative control. On the second day after transfection, puromycin was added into the medium at the concentration of 0.5µg/ml. Then the transfected cells were washed with PBS and changed with fresh medium with 0.5µg/ml puromycin every three days. After all the negative control cells died, all the library-transfected cells that still attached to the wells were collected by scraping into ice-cold TBS buffer.

Rescue of library plasmids was carried out by the extraction of total DNA from the collected puromycin resistant cells according to Blin and Stafford's method. The extracted DNA was then directly electroporated into the competent cell XL1-blue and plated on LB plates with ampicillin. All the colonies were picked up for plasmid preparation. The plasmids were first screened by size using agarose gel electrophoresis to get rid of the reporters. The rest of the plasmids were sequenced by ALF express DNA automatic sequencing system (Pharmacia) using EBV Reverse sequencing primer (Invitrogen) to reveal the DNA sequence, hence amino acid sequence of the selected peptide ligands.

### Results

No pCEP4-transfected cells survived in the well after 7 days of selection with puromycin, while there were still some living library-transfected cells sticking to the bottom of the well.

Electroporation of the DNA extracted from the library-transfected cells after 7 days of selection gave ~150 colonies. Among them, 50 of the colonies have the right size of library plasmid and were sequenced. One ligand peptide sequence was found. The DNA and amino acid sequences are:

DNA: 5'-GGTACCAAGAAGGAGTTTACATATG GGA TGG TGT ACT TGA TAA
KpnI
Amino acid: M G W C T stop stop

DNA: GGATCCAAGCTT-3'
HindIII

### D - Validation of the selected peptide ligand

The purpose of this experiment is to confirm that the selected peptide ligand indeed restores the mutated p53 protein to the wild-type p53 function in SW480.7 cells. The selection of peptide ligands was based on puromycin resistance. There might be a chance that the cells got the resistance with, or even without the help of the selected ligand via mechanisms other than p53-transactivated p21 promoter. Thus we should make sure that the mutant p53 in SW480.7 cells functions as wild-type p53 only if the selected ligand is added. Confirmation/validation was achieved in two different ways:
1- We observed the function of the peptide ligand on cell cycle control of SW480.7 cells, as well as that of U-2 OS (WTp53) and Saos2 cells (no p53 copy). The selected peptide ligand can be further verified only if it can induce cell cycle arrest or apoptosis in SW480.7 cells but neither in U-2 OS nor in Saos2 cells, which means the specificity of the selected ligand to p53 mutation in SW480.7.
2- We also tested the transcription levels of genes that are only transactivated by wild-type p53. If the transcription levels of these genes are highly induced in ligand treated cells compared with the negative control (cells transfected only with the empty vector (pCEP4). The selected ligand/ peptide was proved to be capable of altering the mutant p53 function in SW480.7 into wild-type function.

P53 (wild-type) regulates different cell responses via transactivating different downstream genes. For example p53 causes cell cycle arrest at G1 phase by transactivation of p21 gene. While p53 causes cell arrest at G2 phase by transactivation of CD95 gene. Apoptosis is due to p53 transactivation of 14-3-3σ and/or Bax gene. Thus we measured p21, CD95, and 14-3-3σ gene transcription levels by RT PCR, to reveal the capability of the selected peptide ligand in restoring the wild-type p53 transactivation of the downstream genes. The expression level of β-actin was measured as a control. β-Actin transcription level was used to normalize total mRNA amount between samples for comparison owing to its constitutive expression in all cells.

### Materials and Methods

SW480.7, U-2 OS, Saos 2, DMEM, McCoy's medium, FCS, PBS, trypsin, trypan blue (Sigma), calcium phosphate transfection reagents, peptide ligand (selected above), pCEP4 (Invitrogen), TRIZOL^{®} Reagent, chloroform, isopropyl alcohol, 75% ethanol (in DEPC-treated water), 0.01%(v/v) DEPC water, SuperScripII reverse transcriptase (Life Technology), TE buffer (10 mM Tris (pH7.6), 1 mM EDTA), ethanol, 4M ammonium acetate (pH7.0), 1-kb DNA ladder, 10 x TBE electrophoresis buffer, 1% agarose in 1xTBE buffer with ethidium bromide.
Primers for RT-PCR are listed in Table 3.

**Table 3. RT-PCR primer sequences.**

| Name | Sequence |
|---|---|
| RT-WAF1-FOR | 5'-CTACCTCAGGCAGCTCAAGC-3' |
| RT - HME1 - FOR | 5'-AGACAGCACCCTCATCATGC-3' |
| RT-CD95-FOR | 5'-TGGTGCTCATCTTAATGGCC-3' |
| RT-ACTIN-FOR | 5'-TGACAAAACCTAACTTGCGC-3' |
| CDS Primer | 5'-AAGCAGTGGTAACAACGCAGAGTACT₍₃₀₎N_₁N-3' |
| PCR Primer | 5'-AAGCAGTGGTAACAACGCAGAGT-3' |

Target genes and their corresponding RT-PCR product using the above primers are listed in Table 4.

**Table 4. RT-PCR primer target genes and their corresponding product.**

| Primer name | Target gene | | Amplify region in target mRNA | Size of RT-PCR product |
|---|---|---|---|---|
| | Name | Function | | |
| RTWAF1FOR | p21 | G1 arrest | 1514-2121 | 607 bp |
| RTHME1FOR | 14-3-3σ | G2 arrest | 652-1245 | 593 bp |
| RTCD95FOR | CD95 | Apoptosis | 1968-2534 | 566 bp |
| RTACTINFOR | β-actin | cytoskeleton | 1236-1793 | 557 bp |

Cells SW480.7, U-2OS, and Sa-os2 were transfected with peptide ligand and pCEP4 parallelly using the calcium phosphate method. The transfection plan was shown in Table 5:

**Table 5. Transfection plan for the verification of peptide ligand function.**

| Cell type | SW480.7 | | U-2OS | | Sa-os2 | |
|---|---|---|---|---|---|---|
| DNA | Ligand | pCEP 4 | Ligand | pCEP 4 | Ligand | pCEP4 |
| Number of 6-well plates | 3 | 3 | 1 | 1 | 1 | 1 |

After transfection, cells were cultured without drug selection and diluted at the ratio of 1:100 every five days. Cell proliferation status was closely observed until ligand transfected SW480.7 cells died out. Life staining with 0.4% trypan blue was applied to determine the death of the cells. Dead cells can not exclude the dye thus were stained blue. Living cells can pump the dye outside thus were not stained.

Total RNA was collected from two wells of one ligand transfected SW480.7 plate and one pCEP4 transfected SW480.7 plate each day on day 0, day 1, and day 2 after transfection. Thus RNA samples were harvested as SW480.7/Ligand-0day, SW480.7/Ligand-1day, SW480.7/Ligand-2day, SW480.7/pCEP4-0day, SW480.7/pCEP4-1day, SW480.7/pCEP4-2day. Protocol for total RNA extraction by TRIZOL^{®} Reagent was according to that given by Life Technology:

### 1.Homogenization

Wash the cell with DEPC water once, then add 1ml of TRIZOL Reagent per well. Pass the cell lysate several times through a pipette.

### 2.Phase separation

Incubate the homogenized samples for 5 minutes at room temperature to permit the complete dissociation of nucleoprotein complexes. Add 200 µl of chloroform per 1 ml of TRIZOL Reagent. Cap the sample tubes securely. Shake tubes vigorously by hand for 15 seconds and incubate them at room temperature for 2 to 3 minutes. Centrifuge the samples at no more than 12,000 x g for 15 minutes at 2 to 8°C.

### 3.RNA precipitation

Transfer the aqueous phase to a fresh tube. Precipitate the RNA by adding 500 µl of isopropyl alcohol per 1 ml of TRIZOL Reagent used for the initial homogenization. Incubate samples at room temperature for 10 minutes and centrifuge at no more than 12,000 x g for 10 minutes at 2 to 8°C.

### 4.RNA wash

Remove the supernatant. Wash the RNA pellet once with 75% ethanol, adding at least 1 ml of 75% ethanol per 1 ml of TRIZOL Reagent used for the initial homogenization. Mix the sample by vortexing and centrifuge at no more than 7,500 x g for 5 minutes at 2 to 8°C.

### 5.Re-dissolving the RNA

Briefly dry the RNA pellet. Dissolve RNA in DEPC water by passing the solution several times through a pipette tip, and incubation for 10 minutes at 55 to 60°C. Determine the concentration by measure OD₂₆₀. Store at - 70°C.

### RT-PCR:

### First-Strand cDNA Synthesis

1. For each RNA sample, combine the following reagents in a sterile 0.2-ml reaction tube:

| | |
|---|---|
| 1-3 µl | RNA sample |
| | (using same amount of RNA among samples) |
| 1 µl | CDS primer (10 µM) |
| 1 µl | RT forward primer (10 µM) |
| x µl | Deionized H₂O |
| 5 µl | Total volume |

2. Mix and spin the tube briefly.
3. Incubate the tube at 70°C in a thermal cycler for 2 min.
4. Spin the tube briefly to collect contents at the bottom. Keep tube at room temperature.
5. Add the following to each reaction tube:

| | |
|---|---|
| 2 µl | 5 x First-Strand Buffer |
| 1 µl | DTT (20 mM) |
| 1 µl | 50 x dNTP (10 mM) |
| 1 µl | SuperScripII reverse transcriptase (200 units/µl) |

6. Gently vortex and spin the tubes briefly.
7. Incubate the tubes at 42°C for 1 hr in an air incubator or cycler.
8. Add 40 µl TE buffer (10 mM Tris (pH7.6), 1 mM EDTA) to dilute the first-strand reaction product.
9. Heat tubes at 72°C for 7 min to inactivate reverse transcriptase.
10. Samples can be stored at -20°C for up to three months.

### cDNA Amplification by PCR

1. Preheat the PCR thermal cycler to 95°C.
2. For each reaction, 5 µl of diluted first-strand cDNA and 5 µl of deionized water are added to a. labeled 0.2-ml reaction tube.
3. Prepare a master mix for all reaction tubes, plus one additional tube. For each reaction:

| | |
|---|---|
| 20.5 µl | Deionized water |
| 5 µl | 10 x PCR buffer |
| 1 µl | 50 x dNTP (10 mM) |
| 1. 5 µl | PCR primer (10 µM) |
| 1.5 µl | RT forward primer (10 µM) |
| 10 µl | 5x Q solution |
| 0.5 µl | HotStart Taq DNA polymerase |
| 40 µl | Total volume |

4. Mix by vortexing and spin the tube briefly.
5. Aliquot 40 µl of the PCR Master Mix into each tube from Step 2.
6. Cap the tube, and place it in the preheated thermal cycler.
7. PCR was carried out using the following cycling parameters:

| | |
|---|---|
| 95 °C | 15 min |
| 2 cycles | |
| 94 °C | 30 sec |
| 70 °C | 30 sec |
| 72 °C | 1 min |
| 2 cycles | |
| 94 °C | 30 sec |
| 69 °C | 30 sec |
| 72 °C | 1 min |
| 2 cycles | |
| 94 °C | 30 sec |
| 67 °C | 30 sec |
| 72 °C | 1 min |
| 2 cycles | |
| 94 °C | 30 sec |
| 65 °C | 30 sec |
| 72 °C | 1 min |
| 2 cycles | |
| 94 °C | 30 sec |
| 63 °C | 30 sec |
| 72 °C | 1 min |
| 2 cycles | |
| 94 °C | 30 sec |
| 61 °C | 30 sec |
| 72 °C | 1 min |
| 2 cycles | |
| 94 °C | 30 sec |
| 59 °C | 30 sec |
| 72 °C | 1 min |
| 2 cycles | |
| 94 °C | 30 sec |
| 57 °C | 30 sec |
| 72 °C | 1 min |
| 40 cycles | |
| 94 °C | 30 sec |
| 55 °C | 30 sec |
| 72 °C | 1 min |
| 1 cycle | |
| 72 °C | 7 min |
| 4 °C | store |

8. Electrophoreses 5 µl of each PCR reaction alongside 0.1 µg of 1-kb DNA ladder on a 1.2% agarose/EtBr gel in 1 x TBE buffer.

### Results

After transfecting the ligand into SW480.7 cells for 15 days without puromycin selection, the cells could no longer attach to the wall and died. On the contrary, the ligand-transfected U2-OS and Saos-2 cells were growing well.

RT-PCR analysis of the mRNA from ligand transfected SW480.7 cells showed that this ligand can reestablish wild type p53 function. Higher mRNA levels of p21 (WAF1/Cip1), 14-3-3σ and CD95 genes were found compared with that in the empty pCEP4 transfected SW480.7 cells. This proved that the apoptosis and cell growth arrest of ligand transfected cells were owing to the ligand that corrected R273H/P309S p53 mutant to wild type function.

### Example 3 - New reporter system utilising the Bax promoter

The previous p53 reporter p21ur is based on the transactivation activity of wild type p53 to p21 promoter. It has been proved that up-regulation of p21 protein expression will result in cell growth arrest, but not apoptosis. Another p53 target, Bax (a cell death gene), can lead to apoptosis once its transcription and expression is upregulated. Bax is one of the key mediators for p53 to induce cell apoptosis - to kill the cells that have failed to repair their damaged genomes.

Bax promoter is different from p21 promoter, though both have p53 consensus binding sequence. It has been reported that some p53 mutant can transactivate p21 promoter, but not Bax. This indicates a more stringent requirement of p53 conformation for the transactivation of Bax promoter.

Green fluorescence protein (GFP) has also been used in the new reporter system. Bax promoter drives a long transcript of both puromycin resistant gene and GFP gene. By inserting an IRES fragment between the two genes that introduces a second ribosome-binding site, they can be translated into protein simultaneously from one transcript (Clontech pIRESneo manual). Thus the green color from GFP will visualize the Bax promoter activity, hence the ligand's activity to correct p53 mutants, in addition to the positive selection of puromycin resistance. The green fluorescence will eliminate the background caused by mechanisms which have cells developed to survive from puromycin selection other than transactivation of Bax promoter.

Selection marker is important for integration of the plasmid into genome. A stable p53-null cell line with integrated reporter system will be convenient for ligand screening, especially for non-peptide ligands.

### Materials and Methods

Plasmids used in this Example are: p21ur (above), pIRESneo (Clontech), pEGFP-C1 (Clontech), and pREP8 (Invitrogen).

### Genomic DNA (human, male, normal)

Restriction endonucleases (Promega): AgeI, EcoRV, HindIII, NruI, PvuI, SacI, SalI, SpeI, XbaI, XhoI, XmaI.

Modification enzymes: Vent DNA polymerase (NEB), T4 DNA ligase (Promega), and Shrimp alkaline phosphotase (SAP).

### Bax promoter primer:

Bax promoter Forward:

5'-atctaagcttgaggcttcagcccgggaattccag-3'
HindIII

Bax promoter Reverse:

5'-atctaccggtgccagcagtggcgccgtccaacag-3'
AgeI

### EGFP primer:

EGFP Forward:

5'-aataacccgGGTCGCCACCATGGTGAGCAAG-3'
XmaI

EGFP Reverse:

5'-aataatctagaACTTGTACAGCTCGTCCATGCCG-3'
XbaI
stop

Construction of pBaxur - Bax promoter controlled puromycin resistant gene:
- Making PCR from normal human genomic DNA using Bax promoter primers. The PCR product is 500bp. It has a SmaI site near its 5'-end and a SacI site near its 3'-end.
- Cut PCR product Bax promoter with HindIII and AgeI.
- Cut p21ur with HindIII and AgeI, and dephosphorylate afterwards. Purify the 3,9kb fragment from gel.
- Ligate Bax promoter into the 3,9kb fragment so that Bax promoter can control the puromycin resistant gene expression in mammalian cells. The final product is denoted as pBaxur.

Construction of pBaxurEGFP and p21urEGFP - Green fluorescent protein downstream of puromycin resistant gene that visualizes the transcription level of Bax or p21 promoter:
- Amplify EGFP gene fragment from pEGFP-C1 vector by PCR with the EGFP primers.
- The PCR product is around 750bp. Cut the product with XmaI and XbaI, then purify it from gel.
- Cut pIRESneo with XmaI and XbaI, then purify the 4,5kb fragment from gel.
- Ligate PCR product with pIRESneo 4,5kb fragment to generate vector pIRES-EGFP (5,2kb).
- Cut p21ur or pBaxur with EcoRV and XbaI, and purify the 3.2kb or 1.3kb fragment from gel.
- Cut pIRES-EGFP with NruI and SpeI, dephosphorylate, and purify the 4.4kb fragment from gel.
- Ligate the two fragments to generate transient p53-double-reporter p21urEGFP (7.6kb) or pBaxurEGFP (5.7kb).

Construction of pBaxurEGFP-hisD - Integrative plasmid with selection mark as histidinol D resistant:
- Cut pREP8 with PvuI, SalI, and SacI, purify the 5194bp fragment from gel.
- Cut pBaxEGFP with PvuI and XhoI, dephosphorylated, and purify the 3972bp fragment from gel.
- Ligate the hisD fragment into pBaxurEGFP fragment to generate integrative p53-double reporter pBaxurEGFP_hisD.

The structure of p21urEGFP was verified by restriction of HindIII, which should give five bands of 3489bp, 2339bp, 1041bp, 360bp, and 348bp. Agarose gel electrophoresis of the restricted plasmid showed the expected bands.

The structure of pBaxur was verified by restriction of either HindIII together with SacI, which should give two bands of 3996bp, and 388bp, or SmaI alone, which should give three bands of 3184bp, 620bp, and 580bp. Agarose gel electrophoresis of the restricted plasmid showed the expected bands.

The structure of pBaxurEGFP was verified by restriction of either EcoRI together with XbaI, which should give two bands of 2907bp, and 2766bp, or SacI alone, which should give two bands of 3125bp, and 2548bp. Agarose gel electrophoresis of the restricted plasmid showed the expected bands.

The structure of pBaxurEGFP-hisD was verified by restriction of either EcoRI, which should give two bands of 5955bp, and 3211bp, or SacI, which should give two bands of 6618bp, and 2548bp. Agarose gel electrophoresis of the restricted plasmid showed the expected bands.

Screening for new peptide ligands is done by first transfecting the outlined new reporter p21urEGFP-hisD along with the cotransfection of the peptide library constructed above into cancer cell lines such as SW480.7. Caspase inhibitor VAD-fmk (ApoAlert^{®}, Clontech) is added to the cell culture at the final concentration of 40mM in order to prevent apoptisis in the case of the overexpression of BAX gene. Also 0.5µg/ml puromycin is added in the media one-day after transfection. After several days, surviving green cells (excited at 488nm wavelength) will be the cells containing the cadidate ligands. Rescued plasmids from these cells are subjected to DNA sequencing to deduce the amino acid sequences of the encoded peptide ligands.

When screening non-peptide ligands, a stable cell line of the reporter p21urEGFP-hisD is constructed. Members of the chemical compound library are added to the cell culture. The cells should also be cultured in the medium containing 40mM VAD-fmk and 0.5µg/ml puromycin. Surviving green cells after excitation at 488nm is the indicator for successfull candidate ligands.

## Claims

1. A method of screening a library of peptides of formula M-G/M/V- (X)ₙ wherein n is an integer from 3 to 18, M is methionine, G is glycine, V is valine and each X, which may be the same or different, is any genetically encoded amino acid, which method comprises:
a) transforming a host cell population with a library of nucleic acid constructs which can express free peptides of formula M-G/M/V- (X)ₙ, said free peptides are optionally initially expressed as a fusion peptide with a cleavable signal sequence responsible for localization or translocation of the peptide of formula M-G/M/V- (X)ₙ, said signal sequence being no more than 30 amino acids in length;
b) culturing the transformed host cells under conditions suitable for intra-cellular expression of the peptides of formula M-G/M/V- (X)ₙ; and
c) analysing the host cell population to determine the effect of the peptides of formula M-G/M/V- (X)ₙ on a reporter system, wherein the reporter system includes a target protein and the ability of said peptides to restore the function of said target protein is analysed.

2. A method of identifying a peptide ligand of formula M-G/M/V- (X)ₙ as defined in claim 1 having the ability to restore the function of a target protein, which method comprises:
a) transforming a host cell population with a library of nucleic acid constructs which can express free peptides of formula M-G/M/V- (X)ₙ, said free peptides are optionally initially expressed as a fusion peptide with a cleavable signal sequence responsible for localization or translocation of the peptide of formula M-G/M/V- (X)ₙ, said signal sequence being no more than 30 amino acids in length;
b) culturing the transformed host cells under conditions suitable for intra-cellular expression of the peptides of formula M-G/M/V- (X)ₙ;
c) analysing the host cell population to determine the effect of the expressed peptides on said target protein, which target protein forms part of a reporter system; and optionally
d) identifying the peptide of formula M-G/M/V- (X)ₙ in those cells in which the reporter system indicates a positive response.

3. A method as claimed in claim 1 or claim 2 wherein n is an integer from 3 to 5.

4. A method as claimed in any one of claims 1 to 3 wherein the target protein is selected from a transcription factor, an enzyme, a peptide hormone and a receptor molecule.

5. A method as claimed in any one of claims 1-4 wherein the target protein is a nucleic acid binding protein.

6. A method as claimed in claim 5 wherein the nucleic acid binding protein is p53.

7. A method as claimed in any preceding claim wherein the reporter system comprises a reporter gene.

8. A method as claimed in claim 7 wherein the reporter gene is operably linked to a sequence of nucleotides which provides a binding site for the target protein or for a protein which associates with or is a substrate for the target protein.

9. A method as claimed in claim 8 wherein the reporter gene is operably linked to a p21 or Bax promoter.

10. A method as claimed in any one of claims 7 to 9 wherein the protein product of the reporter gene includes a secretion signal peptide.

11. A method as claimed in any one of claims 7 to 10 wherein the protein product of the reporter gene includes a transmembrane domain.

12. A method as claimed in any one of claims 7 to 11 wherein the host cells have been transferred with said reporter gene.

13. A method as claimed in any preceding claim wherein the peptide library has at least 500 different members.

14. A method as claimed in any preceding claim wherein the host cells are eukaryotic cells.

15. A library of nucleic acid constructs which each express free peptides in an intra-cellular environment, each of said peptides consisting of the sequence M-G/M/V-(X)ₙ, wherein n is an integer from 3 to 18, M is methionine, G is glycine, V is valine and each X, which may be the same or different, is any genetically encoded amino acid, said free peptides are optionally initially expressed as a fusion peptide with a cleavable signal sequence responsible for localization or translocation of the peptide of formula M-G/M/V- (X)ₙ, said signal sequence being no more than 30 amino acids in length and wherein said library has at least 2000 different members.

16. A library as claimed in claim 15 wherein each nucleic acid construct contains a promoter region which is operably linked to the sequence which encodes the peptide of formula M-G/M/V-(X)ₙ.

17. A library as claimed in claim 15 or 16 wherein the nucleic acid constructs are in the form of expression vectors.

18. A library as claimed in claim 17 wherein the expression vectors are capable of autonomous replication.

19. A library of free peptides, each member of said library having the amino acid sequence M-G/M/V- (X)ₙ, wherein n is an integer from 3 to 18, M is methionine, G is glycine, V is valine and each X, which may be the same or different, is any genetically encoded amino acid, each free peptide optionally forming part of a fusion peptide with a cleavable signal sequence responsible for localization or translocation of the peptide of formula M-G/M/V- (X)ₙ, said signal sequence being no more than 30 amino acids in length and wherein said library has at least 2000 different members.

20. A library as claimed in any one of claims 15 to 19 wherein n is an integer from 3 to 10.

21. A library as claimed in claim 20 wherein n is an integer from 3 to 5.

22. A library as claimed in any one of claims 15 to 21 wherein the value of n is the same for each member of the library.

23. A method of generating a library of nucleic acid constructs which each express free peptides in an intra-cellular environment, said free peptides consisting of the formula M-G/M/V- (X)ₙ wherein n is an integer from 3 to 18, M is methionine, G is glycine, V is valine and each X, which may be the same or different, is any genetically encoded amino acid, said free peptides optionally being initially expressed as a fusion peptide with a cleavable signal sequence responsible for localization or translocation of the peptide of formula M-G/M/V- (X)ₙ, said signal sequence being no more than 30 amino acids in length, which method comprises synthesising a library of DNA molecules which each include the nucleotide sequence ATGGGA(NNK)ₙ, wherein n is an integer from 3 to 18, N is A, C, T or G and K is G or T and wherein each NNK triplet may be the same or different, and inserting a library of synthesised DNA fragments which each includes a nucleotide sequence of formula ATGGGA(NNK)ₙ into expression vectors, wherein said library has at least 2000 different members.

24. A method as claimed in claim 23 wherein during synthesis of the library of DNA molecules equimolar amounts of nucleotides A, C, T and G are added for incorporation at each N position and equimolar amounts of nucleotides G and T are added for incorporation at each K position.

25. A library of nucleic acid constructs prepared according to a method of claim 23 or claim 24, wherein said library has at least 2000 different members.

## Patentansprüche

1. Verfahren zum Screenen einer Bibliothek von Peptiden der Formel M-G/M/V- (X)ₙ, wobei n eine ganze Zahl von 3 bis 18 ist, M Methionin ist, G Glycin ist, V Valin ist und jedes X, das gleich oder verschieden sein kann, irgendeine genetisch codierte Aminosäure ist, wobei das Verfahren umfasst:
a) Transformieren einer Wirtszellenpopulation mit einer Bibliothek von Nukleinsäurekonstrukten, die freie Peptide der Formel M-G/M/V- (X)ₙ exprimieren können, wobei die freien Peptide wahlweise anfänglich als Fusionspeptid mit einer spaltbaren Signalsequenz exprimiert werden, die für die Lokalisierung oder Translokation des Peptids der Formel M-G/M/V- (X)ₙ verantwortlich ist, wobei die Signalsequenz nicht mehr als 30 Aminosäuren lang ist;
b) Kultivieren der transformierten Wirtszellen unter Bedingungen, die für die intrazelluläre Expression der Peptide der Formel M-G/M/V- (X)ₙ geeignet sind; und
c) Analysieren der Wirtszellenpopulation, um die Wirkung der Peptide der Formel M-G/M/V- (X)ₙ auf ein Reportersystem zu bestimmen, wobei das Reportersystem ein Zielprotein aufweist und die Fähigkeit der Peptide, die Funktion des Zielproteins wiederherzustellen, analysiert wird.

2. Verfahren zum Identifizieren eines Peptidliganden der Formel M-G/M/V- (X)ₙ nach Anspruch 1 mit der Fähigkeit, die Funktion eines Zielproteins wiederherzustellen, wobei das Verfahren umfasst:
a) Transformieren einer Wirtszellenpopulation mit einer Bibliothek von Nukleinsäurekonstrukten, die freie Peptide der Formel M-G/M/V- (X)ₙ exprimieren können, wobei die freien Peptide wahlweise anfänglich als Fusionspeptid mit einer spaltbaren Signalsequenz exprimiert werden, die für die Lokalisierung oder Translokation des Peptids der Formel M-G/M/V- (X)ₙ verantwortlich ist, wobei die Signalsequenz nicht mehr als 30 Aminosäuren lang ist;
b) Kultivieren der transformierten Wirtszellen unter Bedingungen, die für die intrazelluläre Expression der Peptide der Formel M-G/M/V- (X)ₙ geeignet sind;
c) Analysieren der Wirtszellenpopulation, um die Wirkung der exprimierten Peptide auf das Zielprotein zu bestimmen, wobei das Zielprotein einen Teil eines Reportersystems bildet; und wahlweise
d) Identifizieren des Peptids der Formel M-G/M/V- (X)ₙ in jenen Zellen, in denen das Reportersystem auf eine positive Reaktion hinweist.

3. Verfahren nach Anspruch 1 oder Anspruch 2, wobei n eine ganze Zahl von 3 bis 5 ist.

4. Verfahren nach einem der Ansprüche 1 bis 3, wobei das Zielprotein aus einem Transkriptionsfaktor, einem Enzym, einem Peptidhormon und einem Rezeptormolekül ausgewählt ist.

5. Verfahren nach einem der Ansprüche 1-4, wobei das Zielprotein ein Nukleinsäure-Bindungsprotein ist.

6. Verfahren nach Anspruch 5, wobei das Nukleinsäure-Bindungsprotein p53 ist.

7. Verfahren nach einem vorangehenden Anspruch, wobei das Reportersystem ein Reportergen aufweist.

8. Verfahren nach Anspruch 7, wobei das Reportergen mit einer Sequenz von Nukleotiden funktionsfähig verbunden ist, die eine Bindungsstelle für das Zielprotein oder für ein Protein, das sich mit dem Zielprotein zusammenschließt oder ein Substrat dafür ist, bereitstellt.

9. Verfahren nach Anspruch 8, wobei das Reportergen mit einem p21- oder Bax-Promotor funktionsfähig verbunden ist.

10. Verfahren nach einem der Ansprüche 7 bis 9, wobei das Proteinprodukt des Reportergens ein Sekretionssignalpeptid aufweist.

11. Verfahren nach einem der Ansprüche 7 bis 10, wobei das Proteinprodukt des Reportergens eine Transmembrandomäne aufweist.

12. Verfahren nach einem der Ansprüche 7 bis 11, wobei die Wirtszellen mit dem Reportergen übertragen wurden.

13. Verfahren nach einem vorangehenden Anspruch, wobei die Peptidbibliothek mindestens 500 verschiedene Mitglieder aufweist.

14. Verfahren nach einem vorangehenden Anspruch, wobei die Wirtszellen eukaryotische Zellen sind.

15. Bibliothek von Nukleinsäurekonstrukten, die jeweils freie Peptide in einer intrazellulären Umgebung exprimieren, wobei jedes der Peptide aus der Sequenz M-G/M/V- (X)ₙ besteht, wobei n eine ganze Zahl von 3 bis 18 ist, M Methionin ist, G Glycin ist, V Valin ist und jedes X, das gleich oder verschieden sein kann, irgendeine genetisch codierte Aminosäure ist, wobei die freien Peptide wahlweise anfänglich als Fusionspeptid mit einer spaltbaren Signalsequenz exprimiert werden, die für die Lokalisierung oder Translokation des Peptids der Formel M-G/M/V- (X)ₙ verantwortlich ist, wobei die Signalsequenz nicht mehr als 30 Aminosäuren lang ist und wobei die Bibliothek mindestens 2000 verschiedene Mitglieder aufweist.

16. Bibliothek nach Anspruch 15, wobei jedes Nukleinsäurekonstrukt einen Promotorbereich enthält, der mit der Sequenz funktionsfähig verbunden ist, die das Peptid der Formel M-G/M/V- (X)ₙ codiert.

17. Bibliothek nach Anspruch 15 oder 16, wobei die Nukleinsäurekonstrukte in Form von Expressionsvektoren vorliegen.

18. Bibliothek nach Anspruch 17, wobei die Expressionsvektoren zur autonomen Replikation in der Lage sind.

19. Bibliothek von freien Peptiden, wobei jedes Mitglied der Bibliothek die Aminosäuresequenz M-G/M/V- (X)ₙ aufweist, wobei n eine ganze Zahl von 3 bis 18 ist, M Methionin ist, G Glycin ist, V Valin ist und jedes X, das gleich oder verschieden sein kann, irgendeine genetisch codierte Aminosäure ist, wobei jedes freie Peptid wahlweise einen Teil eines Fusionspeptids mit einer spaltbaren Signalsequenz bildet, die für die Lokalisierung oder Translokation des Peptids der Formel M-G/M/V- (X)ₙ verantwortlich ist, wobei die Signalsequenz nicht mehr als 30 Aminosäuren lang ist und wobei die Bibliothek mindestens 2000 verschiedene Mitglieder aufweist.

20. Bibliothek nach einem der Ansprüche 15 bis 19, wobei n eine ganze Zahl von 3 bis 10 ist.

21. Bibliothek nach Anspruch 20, wobei n eine ganze Zahl von 3 bis 5 ist.

22. Bibliothek nach einem der Ansprüche 15 bis 21, wobei der Wert von n für jedes Mitglied der Bibliothek gleich ist.

23. Verfahren zum Erzeugen einer Bibliothek von Nukleinsäurekonstrukten, die jeweils freie Peptide in einer intrazellulären Umgebung exprimieren, wobei die freien Peptide aus der Formel M-G/M/V- (X)ₙ bestehen, wobei n eine ganze Zahl von 3 bis 18 ist, M Methionin ist, G Glycin ist, V Valin ist und jedes X, das gleich oder verschieden sein kann, irgendeine genetisch codierte Aminosäure ist, wobei die freien Peptide wahlweise anfänglich als Fusionspeptid mit einer spaltbaren Signalsequenz exprimiert werden, die für die Lokalisierung oder Translokation des Peptids der Formel M-G/M/V- (X)ₙ verantwortlich ist, wobei die Signalsequenz nicht mehr als 30 Aminosäuren lang ist, wobei das Verfahren das Synthetisieren einer Bibliothek von DNA-Molekülen, die jeweils die Nukleotidsequenz ATGGGA(NNK)ₙ aufweisen, wobei n eine ganze Zahl von 3 bis 18 ist, N A, C, T oder G ist und K G oder T ist, und wobei jedes NNK-Triplett gleich oder verschieden sein kann, und das Einfügen einer Bibliothek von synthetisierten DNA-Fragmenten, die jeweils eine Nukleotidsequenz der Formel ATGGGA(NNK)ₙ aufweisen, in Expressionsvektoren umfasst, wobei die Bibliothek mindestens 2000 verschiedene Mitglieder aufweist.

24. Verfahren nach Anspruch 23, wobei während der Synthese der Bibliothek von DNA-Molekülen äquimolare Mengen von Nukleotiden A, C, T und G für den Einbau in jeder N-Position hinzugefügt werden und äquimolare Mengen von Nukleotiden G und T für den Einbau in jeder K-Position hinzugefügt werden.

25. Bibliothek von Nukleinsäurekonstrukten, die gemäß einem Verfahren nach Anspruch 23 oder 24 hergestellt sind, wobei die Bibliothek mindestens 2000 verschiedene Mitglieder aufweist.

## Revendications

1. Procédé de criblage d'une banque de peptides de formule M-G/M/V- (X)ₙ dans laquelle n est un nombre entier de 3 à 18, M représente méthionine, G représente glycine, V représente valine et chaque X, qui peut être le même ou différent, est n'importe quel acide aminé codé génétiquement, lequel procédé comprend les opérations consistant :
a) à transformer une population de cellules hôtes à l'aide d'une banque de constructions d'acide nucléique qui peut exprimer des peptides libres de formule M-G/M/V- (X)ₙ, lesdits peptides libres étant facultativement initialement exprimés sous forme d'un peptide de fusion avec une séquence signal clivable responsable d'une localisation ou translocation du peptide de formule M-G/M/V- (X)ₙ, ladite séquence signal n'étant pas de plus de 30 acides aminés en longueur ;
b) à réaliser une culture des cellules hôtes, transformées, dans des conditions convenables pour une expression intracellulaire des peptides de formule M-G/M/V- (X)ₙ ; et
c) à analyser la population de cellules hôtes afin de déterminer l'effet des peptides de formule M-G/M/V- (X)ₙ, sur un système rapporteur, et dans lequel le système rapporteur comprend une protéine cible et l'aptitude desdits peptides à restaurer la fonction de ladite protéine cible est analysée.

2. Procédé d'identification d'un ligand peptidique de formule M-G/M/V- (X)ₙ telle que définie à la revendication 1, ayant l'aptitude à restaurer la fonction d'une protéine cible, lequel procédé comprend les opérations consistant :
a) à transformer une population de cellules hôtes à l'aide d'une banque de constructions d'acide nucléique qui peut exprimer des peptides libres de formule M-G/M/V- (X)ₙ, lesdits peptides libres étant facultativement initialement exprimés sous forme d'un peptide de fusion avec une séquence signal clivable responsable d'une localisation ou translocation du peptide de formule M-G/M/V- (X)ₙ, ladite séquence signal n'étant pas de plus de 30 acides aminés en longueur ;
b) à réaliser une culture des cellules hôtes, transformées, dans des conditions convenables pour une expression intracellulaire des peptides de formule M-G/M/V- (X)ₙ ;
c) à analyser la population de cellules hôtes afin de déterminer l'effet des peptides, exprimés, sur ladite protéine cible, laquelle protéine cible fait partie d'un système rapporteur ; et facultativement
d) à identifier le peptide de formule M-G/M/V- (X)ₙ dans celles des cellules dans lesquelles le système rapporteur indique une réponse positive.

3. Procédé tel que revendiqué à la revendication 1 ou à la revendication 2, selon lequel n est un nombre entier de 3 à 5.

4. Procédé tel que revendiqué à l'une quelconque des revendications 1 à 3, selon lequel la protéine cible est choisie parmi un facteur de transcription, une enzyme, une hormone peptidique et une molécule récepteur.

5. Procédé tel que revendiqué à l'une quelconque des revendications 1 à 4, selon lequel la protéine cible est une protéine de liaison d'acide nucléique.

6. Procédé tel que revendiqué à la revendication 5, selon lequel la protéine de liaison d'acide nucléique est p53.

7. Procédé tel que revendiqué à l'une quelconque des revendications précédentes, selon lequel le système rapporteur comprend un gène rapporteur.

8. Procédé tel que revendiqué à la revendication 7, selon lequel le gène rapporteur est lié de manière à pouvoir agir à une séquence de nucléotides qui fournit un site de liaison pour la protéine cible ou pour une protéine qui s'associe à, ou est un substrat pour, la protéine cible.

9. Procédé tel que revendiqué à la revendication 8, selon lequel le gène rapporteur est lié de manière à pouvoir agir à un promoteur p21 ou Bax.

10. Procédé tel que revendiqué à l'une quelconque des revendications 7 à 9, selon lequel le produit protéine du gène rapporteur comprend un peptide signal de sécrétion.

11. Procédé tel que revendiqué à l'une quelconque des revendications 7 à 10, selon lequel le produit protéine du gène rapporteur comprend un domaine transmembranaire.

12. Procédé tel que revendiqué à l'une quelconque des revendications 7 à 11, selon lequel les cellules hôtes ont été transférées avec ledit gène rapporteur.

13. Procédé tel que revendiqué à l'une quelconque des revendications précédentes, selon lequel la banque de peptides possède au moins 500 éléments différents.

14. Procédé tel que revendiqué à l'une quelconque des revendications précédentes, selon lequel les cellules hôtes sont des cellules eucaryotes.

15. Banque de constructions d'acide nucléique qui expriment chacune des peptides libres dans un environnement intracellulaire, chacun desdits peptides consistant en la séquence M-G/M/V- (X)ₙ dans laquelle n est un nombre entier de 3 à 18, M représente méthionine, G représente glycine, V représente valine et chaque X, qui peut être le même ou différent, est n'importe quel acide aminé codé génétiquement, lesdits peptides libres étant facultativement initialement exprimés sous forme d'un peptide de fusion avec une séquence signal clivable responsable d'une localisation ou translocation du peptide de formule M-G/M/V- (X)ₙ, ladite séquence signal n'étant pas de plus de 30 acides aminés en longueur, et dans laquelle ladite banque possède au moins 2000 éléments différents.

16. Banque telle que revendiquée à la revendication 15, dans laquelle chaque construction d'acide nucléique contient une région promoteur qui est liée de manière à pouvoir agir à la séquence qui code le peptide de formule M-G/M/V- (X)ₙ.

17. Banque telle que revendiquée à la revendication 15 ou 16, dans laquelle les constructions d'acide nucléique sont sous la forme de vecteurs d'expression.

18. Banque telle que revendiquée à la revendication 17, dans laquelle les vecteurs d'expression sont capables de réplication autonome.

19. Banque de peptides libres, chaque élément de ladite banque possédant la séquence d'acide aminé M-G/M/V - (X)ₙ dans laquelle n est un nombre entier de 3 à 18, M représente méthionine, G représente glycine, V représente valine et chaque X, qui peut être le même ou différent, est n'importe quel acide aminé codé génétiquement, chaque peptide libre faisant facultativement partie d'un peptide de fusion avec une séquence signal clivable responsable d'une localisation ou translocation du peptide de formule M-G/M/V- (X)ₙ, ladite séquence signal n'étant pas de plus de 30 acides aminés en longueur, et dans laquelle ladite banque possède au moins 2000 éléments différents.

20. Banque telle que revendiquée à l'une quelconque des revendications 15 à 19, dans laquelle n est un nombre entier de 3 à 10.

21. Banque telle que revendiquée à la revendication 20, dans laquelle n est un nombre entier de 3 à 5.

22. Banque telle que revendiquée à l'une quelconque des revendications 15 à 21, dans laquelle la valeur de n est la même pour chaque élément de la banque.

23. Procédé de production d'une banque de constructions d'acide nucléique qui expriment chacune des peptides libres dans un environnement intracellulaire, lesdits peptides libres consistant en la formule M-G/M/V- (X)ₙ dans laquelle n est un nombre entier de 3 à 18, M représente méthionine, G représente glycine, V représente valine et chaque X, qui peut être le même ou différent, est n'importe quel acide aminé codé génétiquement, lesdits peptides libres étant facultativement initialement exprimés sous forme d'un peptide de fusion avec une séquence signal clivable responsable d'une localisation ou translocation du peptide de formule M-G/M/V- (X)ₙ, ladite séquence signal n'étant pas de plus de 30 acides aminés en longueur, lequel procédé comprend les opérations consistant à synthétiser une banque de molécules d'ADN qui comportent chacune la séquence de nucléotides ATGGGA(NNK)ₙ, dans laquelle n est un nombre entier de 3 à 18, N représente A, C, T ou G et K représente G ou T et dans laquelle chaque triplet NNK peut être le même ou différent, et à insérer dans des vecteurs d'expression une banque de fragments d'ADN de synthèse qui comportent chacun une séquence nucléotidique de formule ATGGGA(NNK)ₙ, et dans lequel ladite banque possède au moins 2000 éléments différents.

24. Procédé tel que revendiqué à la revendication 23, selon lequel, pendant une synthèse de la banque de molécules d'ADN, des quantités équimolaires de nucléotides A, C, T et G sont ajoutées pour incorporation en chaque position N et des quantités équimolaires de nucléotides G et T sont ajoutées pour incorporation en chaque position K.

25. Banque de constructions d'acide nucléique préparée suivant un procédé de la revendication 23 ou de la revendication 24, dans laquelle ladite banque possède au moins 2000 éléments différents.
